# EUROPÄISCHE PATENTSCHRIFT

(11) **EP 1 753 553 B1**
(45) Veröffentlichungstag und Bekanntmachung des Hinweises auf die Patenterteilung: **01.08.2007**
(21) Anmeldenummer: 05751903.5
(22) Anmeldetag: 25.05.2005
(51) Int. Cl.: B09B 3/00, C05F 17/02

(54) **ABFALLBEHANDLUNGSANLAGE**
REFUSE TREATMENT PLANT
STATION DE TRAITEMENT DES DECHETS

(30) Priorität: 28.05.2004 DE 102004026694
(43) Veröffentlichungstag der Anmeldung: 21.02.2007
(73) Patentinhaber: W.L.GORE & ASSOCIATES GMBH, 85640 Putzbrunn (DE)
(72) Erfinder: DEYERLING, Lothar, 83677 Reichersbeuren (DE); BAUER, Ambros, 83627 Warngau (DE)
(86) Internationale Anmeldenummer: PCT/EP2005/005648
(87) Internationale Veröffentlichungsnummer: WO 2005/115646

(56) Entgegenhaltungen:
- WO-A-20/04048719
- DE-A1- 4 231 414
- US-A- 3 895 916

## Beschreibung

Die vorliegende Erfindung betrifft den biologischen aeroben Abbau von organischem Material beispielsweise in Abfall mit biologisch abbaubaren Anteilen, Insbesondere betrifft die Erfindung eine Abfallbehandlungsanlage in geschlossener Bauweise mit einer wasser- und partikeldichten Abdeckplane, welche jedoch luft- und wasserdampfdurchlässig ist.

Auf dem Gebiet der Abfallbehandlung sind verschiedene Vorrichtungen und Verfahren zum Trocknen und zum aeroben Abbau biologischer Bestandteile von festen Siedlungsabfällen bekannt. Ein seit langem bestehendes Problem bei der aeroben Abfallbehandlung ist die dabei auftretende Abluft, die einen hohen Anteil an schädlichen Emissionen wie Aerosole, Staub, Keime, VOCs (volatile organic compounds), und Geruchsstoffe aufweist die gesundheitliche Beinträchtigungen hervorrufen können und regionalen Grenzwertemissionen unterworfen sind. Bei vielen einfachen Anlagen zur Abfallbehandlung gibt es Probleme mit der Keimemissions- und Geruchskontrolle, da alle entstehenden Gase ungehindert in die Umgebungsatmosphäre entweichen können.

Zur Behebung dieser Problematik wird seit einigen Jahren die aerobe Abfallbehandlung in geschlossenen Systemen durchgeführt und beinhaltet das Kompostieren in baulich geschlossenen Gebäuden, wie auch Containern, Rottetunneln oder Boxen. Derartige geschlossene Systeme gelten als luftdicht, da die Abluft im Gebäude verbleibt und von dort gezielt abgesaugt wird. Nachteilig an diesem geschlossenen System sind die hohen Investitionskosten bei der Anschaffung einer solchen Vorrichtung als auch die hohen -Kosten, die zur Abführung und Reinigung der innerhalb des Systems entstehenden Abluft anfallen. Insbesondere die Kosten für die Abluftbehandlung mit Biofiltern oder Biowäschern sind sehr hoch.

Alternativ zu den geschlossenen Anlagen werden seit einigen Jahren großflächige permeable Abdeckplanen eingesetzt, welche aufgeschüttete Abfallmieten vollständig bedecken. Die Permeabilität dieser Abdeckungen ist so gewählt, dass die Abfallmiete aktiv belüftet werden kann und gleichzeitig ein Schutz gegen Keimemission und auch eine Geruchsreduktion gegeben ist. Beispielsweise beschreibt die DE 4231414 C2 eine Abdeckung für eine Kompostmiete mit einem wasserdichten und gasdurchlässigen Membranlaminat. Solche Abdeckungen weisen aber verschiedene Nachteile auf. Zum einen erfolgt das Aufsetzen und das Abdecken der Abfallmieten zeitlich nacheinander, das heißt, die Miete muß erst partiell oder vollständig aufgesetzt sein, bevor die Abdeckplane darübergezogen werden kann. Solange die offene, nicht abgedeckte Miete frei von Luft überströmt wird, führt dies zu unerwünschten Emissionen. Außerdem muss zum Abtragen der Miete die Abdeckung von der Miete entfernt werden, damit die Geräte zum Abtragen freien Zugang zur Abfallmiete haben und keine Schäden an der Abdeckung verursachen. Das Entfernen der Abdeckung führt wiederum zu unerwünschten Emissionen. Häufig muß der angelieferte Abfall eine Vorbehandlung wie Sortieren, Zerkleinern oder Mischen durchlaufen. Nach dieser Vorbehandlung wird der Abfall offen durch das Gelände bis zur Miete transportiert und gibt dabei ebenfalls unerwünschte Emissionen an die Umgebung ab.

Zum anderen ergibt sich durch die aufwendige, zumindest teilweise manuelle Handhabung beim Auflegen und Entfernen der Abdeckplane ein erheblicher Personalaufwand. Die Handhabung der Abdeckung wird durch die hohe Schütthöhe der Miete noch erschwert. Weiterhin müssen Randbeschwerungen zum Niederhalten der Abdeckplane, wie zum Beispiel wassergefüllte Feuerwehrschläuche und Sandsäcke entfernt und später wieder plaziert werden. Auch bei der Verwendung mechanischer Wickelungsmaschinen zum Aufbringen und Abziehen der Abdeckplanen werden vorteilhaft mindestens zwei Personen benötigt. Weiterhin kann es durch das Auf- und Abrollen der Abdeckplane zu Beschädigungen des Planenmaterials kommen, was die Funktionalität der Abdeckplane, insbesondere bei einem Membranmaterial, stark herabsetzt. Auch aus Sicht des Arbeitsschutzes ist das Arbeiten mit Abdeckplanen auf Abfallmieten für das zuständige Personal nicht unproblematisch. Einerseits besteht ein Unfallrisiko beim Betreten und Überschreiten der Miete. Zum anderen hat das Personal bei einer nicht abgedeckten Miete unmittelbaren Kontakt mit dem abzudeckenden Material und den austretenden Emissionen, was ein Gesundheitsrisiko für das Personal darstellt.

In einer Weiterentwicklung werden die Abdeckplanen mit aufblasbaren Stützelementen versehen, welche die Abdeckplane zum Zwecke des Auf- und Abbaus der Rottemiete heben und senken können, ohne dass die Abdeckplane von der Miete entfernt werden muß wie Beispielweise im Dokument WO 2004/048719 A1. Jedoch auch hier bleibt das Problem der austretenden Emissionen bestehen, da während des Auf- und Abbaus der Rottemiete ein freier Zugang zu der Miete bestehen muß und in dieser Zeit schädliche Emissionen ungehindert in die Umgebungsluft gelangen können.

Von der Gesetzgebung als auch nationalen und regionalen Genehmigungsbehörden sind Tendenzen erkennbar, die für die Zukunft geschlossenen Systemen den Vorzug geben werden.

Aufgabe der vorliegenden Erfindung ist deshalb eine verbesserte und kostengünstigere Abfallbehandlungsanlage zum aeroben Abbau von organischen Bestandteilen in Abfall, welche gleichzeitig ein zur Umgebung geschlossenes System darstellt.

Es ist weiterhin Aufgabe der vorliegenden Erfindung eine Abfallbehandlungsanlage unter Verwendung von Mietenabdeckungen bereitzustellen, welche auch beim Auf- und Abbau der Miete schädliche Emissionen in die Umgebung vermeiden.

Erfindungsgemäß wird die Aufgabe durch die Merkmale des Anspruch 1 gelöst. Die abhängigen Ansprüche geben vorteilhafte Ausgestaltungen der Erfindung an.

Die Aufgabe wird gelöst durch eine Abfallbehandlungsanlage mit einer Halle mit mindestens einer Hallenwand, einem Hallendach und einem Zugang zum Einführen und Entfernen von Abfall, einer Be- und Entlüftungsanlage zur Zu- und Abfuhr von Luft in und aus der Halle und mindestens einem an einer Außenseite der Hallenwand angeordneten Abfallbehandlungsraum für den aeroben Abbau der organischen Bestandteil in dem Abfall. Die Halle ist mit dem Abfallbehandlungsraum über eine Hallenwandöffnung verbunden, die zum Einführen des Abfalls aus der Halle in den Abfallbehandlungsraum und zum Entfernen des behandelten Abfalls aus dem Abfallbehandlungsraum in die Halle dient. Der Abfallbehandlungsraum wird durch eine mit einer Hebe- und Senkvorrichtung versehenen Abdeckplane gebildet. Die Abdeckplane weist einen Abdeckplanenrand auf, welcher zur Hallenwand und zum Boden mittels einer Befestigungvorrichtung starr und luftdicht angeordnet ist.

Die erfindungsgemäße Abfallbehandlungsanlage bildet ein geschlossenes System und verknüpft eine geschlossene Halle mit einer Mietenabdeckung zu einem einheitlichen System. Insbesonders ist es mit einer solchen Anlage möglich, den Abfall von der Anlieferung, über seine Vorbehandlung und anschließenden aeroben Behandlung bis zur Auslieferung in zur Umgebung geschlossenen Räumen zu halten, so dass ein Kontakt schädlicher Emissionen mit der Umgebungsluft weitestgehend vermieden ist.

Mit der Anlage wird die aerobe Behandlung aus der Halle in die angrenzenden Abfallbehandlungsräume verlagert. Die Hallenwandöffnung ist verschließbar, beispielsweise eine Tür oder ein Tor trennt den Abfallbehandlungsraum luftdicht von dem Inneren der Halle. Lediglich zum Befüllen oder Entleeren des Abfallbehandlungsraumes wird die Hallenwandöffnung geöffnet, während der gesamten Dauer der Behandlung bleibt die Hallenwandöffnung geschlossen.

Damit kann die Halle wesentlich kleiner dimensioniert und gebaut werden. Die Halle wird nur noch zur Annahme des Abfalls und für gegebenenfalls notwendige Aufbereitungsarbeiten wie beispielsweise Zerkleinern oder Mischen benötigt. Somit sinken die Investitionskosten für die gesamte Anlage, zumal durch eine kleinere Halle weniger Be- und Entlüftungskapazität benötigt wird. Damit verbunden können kleinere und somit kostengünstigere Hallenentlüftungssysteme vorgesehen werden. Ebenso kann ein nachfolgender Biofilter kleiner dimensioniert werden. Da die Menge an zu behandelnder Abluft weit geringer ist als bei den geschlossenen Anlagen im Stand der Technik, reduzieren sich die Investitions- und Betriebskosten der erfindungsgemäßen Anlage. Außerdem weist die Halle eine wesentlich höhere Lebensdauer auf, da sie für die intensiven aeroben Behandlungsprozesse nicht mehr verwendet wird.

In einer Ausführungsform weist die Abdeckplane eine wasserdichte und gasdurchlässige Membrane auf. Die Membrane wirkt als Schutzbarriere nach Innen und nach Außen. Nach Innen schützt die Membrane gegen den Eintritt von Wasser und gegen Austrocknung bei starker Sonneneinstrahlung. Nach Außen schützt die Membrane gegen Keimemissionen und den Austritt von Gerüchen und bewirkt aufgrund seiner Gasdurchlässigkeit, dass das in dem Abfallbehandlungsraum durch aeroben Abbau entstehende CO₂ und Luft durch die Membrane entweichen kann, ohne dass es zu einem Druckaufbau an der Abdeckplane kommt. Die Abluft aus den Abfallbehandlungsräumen muß nicht speziell abgesaugt werden, da diese durch die gasdurchlässige Membrane vorgereinigt in die Umgebung entweicht. Die Porenstruktur der Membrane bewirkt ein Herausfiltern aiier schädlicher Bestandteile aus der Abluft. Vorzugsweise ist die wasserdichte und gasdurchlässige Membrane porös. Aufgrund der porösen Ausgestaltung der Membrane weist die Abdeckplane eine Luftdurchlässigkeit zwischen 3 und 100m³/m²/h bei einer Druckdifferenz von 200Pa auf.

Außerdem weist die Abdeckplane mit der Membrane einen Wasserdampfdurchgangswiderstand von weniger als 20m²Pa/W auf und gewährleistet damit einen hohen Wasserdampfdurchgang durch die Abdeckplane. Dieser geringe Wasserdampfdurchgangswiderstand ermöglicht die Trocknung von nassem Material bzw. den Abtransport entstehenden Prozeßwassers. Ein Verklumpen und Verwässern des Abfalls und damit verbundene Faulprozesse werden verhindert. Der Wasserdampfdurchgang als auch die Luftdurchlässigkeit stellt sicher, dass der zu behandelnde Abfall ausreichend belüftet, d.h. ausreichend mit Sauerstoff versorgt wird und Umsetzungsprodukte ohne Druckaufbau entweichen können. Weiterhin ist die Abdeckplane mit der Membrane gegenüber Wasser bei einem Wassereintrittsdruck von größer 10kPa dicht. Dies gewährleistet einen Schutz vor Durchnässung durch Niederschlagswasser.

Vorzugsweise ist die Membrane mit mindestens einer textilen Schicht verbunden. Die Verwendung eines Textillaminates ist besonders vorteilhaft, da neben der hohen Wasserdichtheit und gleichzeitigen Gasdurchlässigkeit die poröse Membrane für die gleichzeitige Zurückhaltung von Emissionen wie Gerüchen und Keimen besonders geeignet ist.

Die Abdeckplane des Abfallbehandlungsraumes ist mit einer Hebe- und Senkvorrichtung versehen, um die Abdeckplane in ihrer Höhe zu verstellen. Die Höhenverstellung ist notwendig für die Befüllung oder Entleerung des Behandlungsraumes mittels Radlader oder teleskopierbarer Förderbänder, Schubböden oder Kratzböden oder ähnlicher Technologien. Das bedeutet, zum Beladen oder Entleeren des Abfallbehandlungsraumes muß sich die Abdeckplane in einem ausreichend hohen Abstand zum Abfall befinden, damit die Geräte oder Maschinen in den Abfallbehandlungraum fahren können.

Während des aeroben Behandlungsprozesses ist es vorteilhaft, wenn die Abdeckplane direkt auf dem zu behandelnden Abfall aufliegt. Dadurch werden die aeroben Abbauprozesse beschleunigt und der Wasserdampfdurchgang durch die Abdeckplane begünstigt. Aus diesem Grund ist die Abdeckplane während der aeroben Behandlung gesenkt und liegt unmittelbar auf dem Abfall.

Das Heben und Senken der Abdeckplane erfolgt mit Ausnahme des Abdeckplanenrandes. Damit der Abfallbehandlungsraum während des Hebens und Senkens der Abdeckplane ein geschlossenes System bleibt, wird der Abdeckplanenrand zur Halle und zum Boden starr und luftdicht mittels einer Befestigungsvorrichtung angeordnet. Die Befestigungsvorrichtung fixiert den Abdeckplanenrand im Vorderbereich des Abfallbehandlungsraumes an der Außenseite der Hallenwand. Der Abdeckplanenrand in den Seitenbereichen und dem Endbereich des Abfallbehandlungsraumes ist entweder direkt am Boden oder an/auf den Anbauwänden mittels Befestigungsvorrichtung fixiert. In diesem Zusammenhang bedeutet luftdicht, dass keine Abluft aus dem Inneren der Anlage über die Verbindung zwischen Halle und Behandlungsraum einschließlich der Befestigung zum Boden hin entweichen darf. Eine starre Befestigung bedeutet, dass der Abdeckplanenrand unbeweglich an der Hallenwand und dem Boden oder an/auf den Anbauwänden befestigt ist. Während der Hebe- und Senkvorgänge bewegt sich die Abdeckplane, jedoch nicht der Abdeckplanenrand.

In einer Ausführungsform ist eine pneumatische Hebe- und Senkvorrichtung vorgesehen, wobei die pneumatische Hebe- und Senkvorrichtung eine Anzahl von aufblasbaren Schläuche vorsieht. Der gehobene Zustand der Vorrichtung wird durch die mit Luft unter Druck gefüllten Schläuche verwirklicht. Dadurch wird die Abdeckplane angehoben und bildet eine befahrbare Halle oder einen Raum aus, in dem der zu behandelnde Abfall aufgeschüttet oder gestapelt werden kann.

Im gesenkten Zustand der Vorrichtung enthalten die Schläuche im wesentlichen keine Luft, d.h. die Schläuche sind leer und die Abdeckplane bedeckt die Abfallmiete. Somit ruht die Abdeckplane wie eine gewöhnliche Mietenabdeckung auf dem Abfall.

In einer weiteren Ausführungsform sind die Schläuche mit einer Flüssigkeit gefüllt.

Die Befestigungsvorrichtung umfaßt Fixierelemente wie beispielsweise Schrauben, Klemmen, Nägel, Nieten oder Spannvorrichtungen, wobei die Verankerung der Fixierelemente in der Hallenwand und im Boden bzw. in der Anbauwand flächig und ditchtend erfolgt.

Insbesondere ist der Abfallbehandlungsraum partikeldicht gegenüber der Umgebung ausgebildet.

Vorteilhafterweise weist die Befestigungsvorrichtung ein anpassungsfähiges Material auf. Dieses anpassungsfähiges Material ist zwischen dem Abdeckplanenrand und der Hallenwand sowie zwischen dem Abdeckplanenrand und dem Boden oder den Anbauwänden angeordnet. Das Material hat die Funktion Unebenheiten in den Wänden und dem Boden auszugleichen, damit keine Fehlluft über Luftkanäle oder Luftbrücken in den Wänden und im Boden aus dem Inneren der Anlage in die Umgebung dringt. Somit wird die Fehlluftrate der gesamten Anlage herabgesetzt. Als anpassungsfähiges Material kann jedes flexible Material verwendet werden welches leicht verformbar ist. Beispielsweise können elastische Kunststoffe wie Schaumstoffe oder Gummis, textile Materialien wie Gewebe, Gewirke oder Vliese, vorzugsweise wasserdicht beschichtet oder hydrophob ausgerüstet, eingesetzt werden. Das anpassungsfähige Material kann flächig als Streifen oder Band Verwendung finden, auch Dichtschnüre können eingesetzt werden, solange ausreichend Material vorhanden ist um mögliche Kanäle, Rillen, Ritzen, Öffnungen und andere Ausbuchtungen luftdicht zu füllen. Je nach Größe und Tiefe der abzudichtenden Öffnungen sollte das Material einlagig oder mehrlagig eingebracht werden.

In einer Ausführungsform ist ein Klemmrahmen vorgesehen, in welchen der Abdeckplanenrand luftdicht geklemmt ist. Der Klemmrahmen selber ist in eine Hallenwandöffnung luftdicht verklemmt oder mit jeder anderen bekannten Art befestigt, sowie entsprechend am Boden oder an/auf den Anbauwänden befestigt.

Weitere Vorteile und Merkmale der Erfindung werden in der folgenden Beschreibung der Zeichnungen und besonderer Ausführungsformen verdeutlicht.

### Definitionen und Testmethoden:

Be- und Entlüftungsanlage: (Abgasreinigungs)einrichtung zur Emissionsminderung von emissionsrelevanten Luftverunreinigungen im Abgas der biologischen Abfallbehandlungsanlage, insbesondere zur Emissionsbegrenzung für Geruchsstoffe, klimarelevante Gase, organische Stoffe und Stäube und zur Reduzierung lebens- und vermehrungsfähiger Mikroorganismen.

Abfallbehandlungsanlage: Abfallbehandlungsanlage, in der Siedlungsabfälle oder andere Abfälle mit biologisch abbaubaren Anteilen mit biologischen oder einer Kombination von biologischen mit physikalischen Verfahren behandelt werden. Insbesondere gehören dazu Einrichtungen zur biologischen Behandlung der Abfälle unter aeroben Bedingungen.

Emissionen sind die von einer biologischen Abfallbehandlungsanlage ausgehenden Luftverunreinigungen. Die Abgasströme einer Abfallbehandlungsanlage müssen bestimmte Emissionsgrenzwerte einhalten, die beispielsweise für Deutschland in dem Bundes-Immissionsschutzgesetz (TA-Luft) vom 24.Juli 2002 festgelegt sind. Unter Emissionen ist zusammenfassend das Austreten von Stäuben, Aerosolen, Gerüchen, Keimen, Pilzsporen, Samen oder anderen ähnlichen Emissionen zu verstehen.

### Wassereintrittsdrucktest

Der Wassereintrittsdrucktest ist ein hydrostatischer Widerstandstest der im wesentlichen darauf beruht, dass Wasser gegen eine Seite einer Materialprobe gepresst wird und die andere Seite der Materialprobe auf den Durchtritt von Wasser hin beobachtet wird.

Der Wasserdruck wird gemäß einem Testverfahren gemessen, in dem destilliertes Wasser bei 20±2°C auf einer Materialprobe mit einer Fläche von 100cm² zunehmend unter Druck gestellt wird. Der Wasseraufstiegsdruck beträgt 60±3cmH₂O/min. Der Wasserdruck ist dann der Druck, bei dem Wasser auf der anderen Seite der Probe erscheint. Die genaue Vorgehensweise ist in der ISO Norm Nr.811 aus dem Jahre 1981 geregelt. Unter "wasserdicht" ist zu verstehen, dass ein Material einen Wassereintrittsdruck von mindestens 10kPa aushält.

Unter "porös" ist ein Material zu verstehen, welches sehr kleine, mikroskopische Poren durch die innere Struktur des Materials aufweist und die Poren eine miteinander verbundene kontinuierliche Verbindung oder Pfad von einer Oberfläche zur anderen Oberfläche des Materials bilden. Entsprechend den Abmessungen der Poren ist das Material somit durchlässig für Luft und Wasserdampf, flüssiges Wasser kann jedoch nicht durch die Poren gelangen. Die Messung der Porengröße kann mit einem Coulter Porometer^{™} erfolgen, hergestellt bei Coulter Electronics, Inc., Hialeah, Florida. Das Coulter Porometer ist ein Instrument das eine automatische Messung der Porengrößenverteilung in porösen Medien nach der im ASTM Standard E1298-89 beschriebenen Methode ermittelt.
Doch nicht von allen verfügbaren porösen Materialien kann die Porengröße mit dem Coulter Porometer ermittelt werden. In einem solchen Fall kann die Porengrößen auch unter Verwendung eines Mikroskopes wie beispielsweise eines Licht- oder Elektronenmikroskopes, ermittelt werden.
Bei Verwendung einer mikroporösen Membrane hat diese eine durchschnittliche Porengröße zwischen 0,1 und 100µm, vorzugsweise liegt die durchschnittliche Porengröße zwischen 0,2 und 10µm.

### Wasserdampfdurchgangswiderstand Ret

Der Ret-Wert ist eine spezifische Materialeigenschaft von Flächengebilden oder zusammengesetzten Materialien, welche den latenten Wärmeverdampfungsfluß bei einem gleichbleibenden Partialdruckgradienten durch eine vorgegebene Oberfläche bestimmt.
Unter "wasserdampfdurchlässig" wird ein Material definiert, das einen Wasserdampfdurchgangswiderstand Ret von unter 150(m²×Pa)/W aufweist. Vorzugsweise weist das Flächengebilde einen Ret von unter 20 (m²×Pa)/W auf. Die Wasserdampfdurchlässigkeit wird durch das Hohenstein MDM Dry Verfahren gemessen, welches in der Standard-Prüfvorschrift Nr. BPI 1.4 (1987) des Bekleidungsphysiologischen Instituts e.V. Hohenstein beschrieben wird.

### Luftdurchlässigkeit

Die Luftdurchlässigkeit wird in m³/h pro m² Flächengebilde angegeben und wird unter Verwendung eines Luftdurchlässigkeitsprüfgeräts von Textest Instruments (FX 3300), Zürich ermittelt. Die Luftdurchiässigkeit wird in Anlehnung an ISO 9237 (1995) ermittelt.
Als "luftdurchlässig" wird ein Material bezeichnet, das eine Luftdurchlässigkeit zwischen 3 und 100 m³/m²/h bei einer angelegten Druckdifferenz von 200Pa aufweist.

Luftdicht: bedeutet im Rahmen dieser Erfindung, dass an der Verbindungstelle von Abdeckplanenrand und Hallenwand bzw. Abdeckplanenrand und Boden oder Anbauwände keine Luft von Innen nach Außen gelangt. Dies kann im Betrieb der Anlage durch Anlegen eines leichten Überdrucks kontrolliert werden. Eine luftdichte Anlage zeigt keine Nebel- und Dampfschwaden an den zu überprüfenden Stellen. Natürlich ist auch eine Überprüfung mit künstlichen Nebel möglich.
Luftdicht schließt im Rahmen dieser Erfindung partikeldicht mit ein.

Abdeckplanenrand: ist die randseitige Begrenzung der Abdeckplane, verläuft um die gesamte Abdeckplane herum.

Partikeldicht: Als partikeldicht wird Abfallbehandlungsraum bezeichnet, soweit er eine Abscheidung von Partikeln >0.3µm ermöglichen. Unter Partikeln sind luftgetragene Teilchen fester oder flüssiger Form wie beispielsweise Stäube, Sporen, Pollen oder Aerosole zu verstehen.

Die Erfindung soll nun anhand von Zeichnungen näher erläutert werden:
Figur 1 zeigt eine schematische Darstellung der erfindungsgemäßen Abfallbehandlungsanlage.
Figur 1A zeigt eine schematische Vergrößerung des Abfallbehandlungsraumes 16a aus Figur1.
Figur 1B zeigt eine schematische Vergrößerung des Abfallbehandlungsraumes 16b aus Figur 1.
Figur 2 zeigt eine schematische Draufsicht auf die erfindungsgemäße Abfallbehandlungsanlage.
Figur 3 zeigt eine perspektivische Ansicht der Abfallbehandlungsanlage.
Figur 4 zeigt einen Querschnitt durch die Abdeckplane eines Abfallbehandlungsraumes.
Figur 5 zeigt eine Ausgestaltung des Abdeckplanenrandes.
Figur 6 zeigt eine schematische Schnittdarstellung durch die Abfallbehandlungsanlage in Figur 1.
Figur 6A zeigt eine schematische Vergrößerung des Abfallbehandlungsraumes 16a aus Figur 6.
Figur 6B zeigt eine schematische Vergrößerung des Abfallbehandlungsraumes 16b aus Figur 6.
Figur 7 zeigt die vergrößerte Darstellung einer Ausgestaltung der Befestigungsvorrichtung in dem Bereich A von Figur 6.
Figur 8 zeigt die vergrößerte Darstellung einer weiteren Ausgestaltung der Befestigungsvorrichtung in dem Bereich A in Figur 6.
Figur 9 zeigt eine vergrößerte Darstellung einer weiteren Ausgestaltung der Befestigungsvorrichtung in dem Bereich B in Figur 6.
Figur 10 zeigt eine weitere Ausgestaltung der Befestigungsvorrichtung.
Figur 11 und 12 zeigen eine Abfallbehandlungsanlage mit einem Behandlungsraum mit einer mechanischen Höhenverstellkonstruktion.

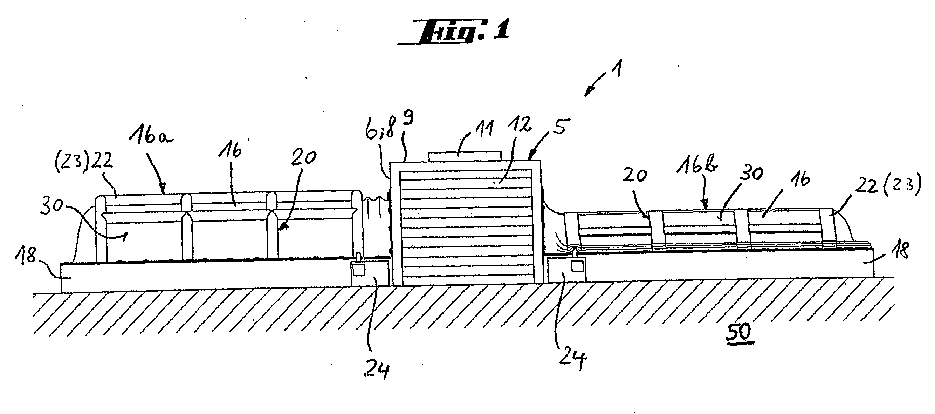

Figur 1 zeigt die Vordersicht einer Abfallbehandlungsanlage (1) gemäß dieser Erfindung. Die Abfallbehandlungsanlage (1) besteht aus einer Halle (5) mit mindestens einer Hallenwand (6), einem Hallendach (9) und einem Zugang (12).

Jeweils an der Außenseite (8) der seitlichen Hallenwände (6) sind eine Anzahl von Abfallbehandlungsräumen (16) angeordnet. In dem Hallendach (9) ist eine Öffnung (11) angeordnet, durch die Luft in das Innere der Halle (5) gelangt. Der Zugang (12) ist die Verbindung zwischen Umgebung und Abfallbehandlungsanlage (1) und vorzugsweise in einer stirnseitigen Hallenwand (6) verschließbar angeordnet. Durch den Zugang (12) wird der Abfall (2) in die Abfallbehandlungsanlage (1) eingeführt und entfernt. Beispielsweise wird der Zugang (12) mittels eines Rolltors oder automatisch sich öffnender und schließender Türen realisiert.
Die Halle (5) dient zur Aufnahme, Vorbehandlung und Verteilung des zu behandelnden Abfalls (2) bevor dieser in den Abfallbehandlungsräumen (16) den eigentlichen aeroben Abbauprozessen unterworfen wird. Gegebenenfalls wird der Abfall (2) in der Halle (5) zerkleinert, gemischt und/oder sortiert. Anschließend wird der Abfall (2) in mindestens einem Abfallbehandlungsraum (16) zu einer Abfallmiete aufgebaut. In jedem Abfallbehandlungsraum (16) findet der aerobe Abbau der organischen Bestandteile des darin aufgeschichteten Abfalls (2) statt. Im Anschluß an den aeroben Behandlungsprozess wird der behandelte Abfall wieder in die Halle (5) und durch den Zugang (12) aus der Halle (5) transportiert.
Es ist auch möglich nur einen Abfallbehandlungsraum (16) an die Hallenwand (6) anzuordnen. In einer Ausführungsform sind insgesamt sechs Abfallbehandlungsräume, jeweils drei nebeneinander, vorgesehen. Die Anzahl der Abfallbehandlungsräume (16) ist beliebig und hängt von der Größe und dem Durchsatz der Abfallbehandungsanlage (1) ab.
Zwischen Halle (5) und Abfallbehandlungsraum (16) ist eine Schleuse in Form einer verschließbaren Öffnung (nicht dargestellt) in der Hallenwand (6) vorgesehen, welche das Innere der Halle (5) mit dem Inneren des Abfallbehandlungsraumes (16) verbindet. Diese Hallenwandöffnung dient zum Einführen des Abfalls (2) aus der Halle (5) in den Abfallbehandlungsraum (16) und zum Entfernen des abgebauten Abfalls aus dem Abfallbehandlungsraum (16) in die Halle (5) und sollte ausreichend groß sein, damit Be- und Entladefahrzeuge oder -geräte wie Radlader, Rollböden, Förderbänder die Öffnung passieren können. Die Hallenwandöffnung ist beispielsweise mittels eines Rolltors verschließbar und soll in der Praxis nur zum Zwecke des Be- und Entladens des Abfallbehandlungsraumes (16) geöffnet sein. Damit können die aeroben Abbauprozesse in dem Abfallbehandlungsraum optimal ablaufen und schädliche Emissionen können nicht aus dem Abfallbehandlungsraum (16) in die Halle (5) gelangen. Vorzugsweise ist die Öffnung luftdicht gegen die Halle (5) verschließbar. In einer Ausführungsform sind Anbauwände (18) vorgesehen, welche den einzelnen Abfallbehandlungsraum (16) seitlich begrenzen. Die Anbauwände (18) sind auf dem Boden (50) errichtet und haben eine maximale Höhe von 2.50m bis 3m. Vorzugsweise beträgt die Höhe der Anbauwände (18) 2m bis 2.50m.
Die Abfallbehandlungsräume (16) weisen jeweils eine Abdeckplane (30) auf, die mit einer Hebe- und Senkvorrichtung (20) versehen ist. In einer Ausführung wird die Hebe- und Senkvorrichtung (20) mittels einer pneumatischen Stützkonstruktion (22), beispielsweise aus aufblasbaren Schläuchen (23) gebildet.
In dem Abfallbehandlungsraum (16) findet der aerobe Behandlungsprozess statt. Dazu ist der Abfallbehandlungsraum (16b) in einem abgesenkten Zustand, das heißt die Hebe- und Senkvorrichtung (20) ist abgesenkt, sodass die daran befestigte Abdeckplane (30) direkt auf dem zu behandelnden Abfall liegt. An der gegenüberliegenden Hallenwand (6) ist der Abfallbehandlungsraum (16a) in einem aufgestellten Zustand dargestellt, das heißt die Hebe- und Senkvorrichtung (20) und die daran befestigte Abdeckplane (30) sind angehoben. Durch den Hebevorgang bildet sich ein Raum aus, welcher für Be- und Entladegeräte oder Be- und Entladefahrzeuge zugänglich ist. Somit kann durch die Hallenwandöffnung Abfall (2) in den Raum transportiert oder aus dem Raum heraus transportiert werden.
Die Abdeckplane (30) hat einen Abdeckplanenrand (32), welcher an der Hallenwand (6) und auf den Abfallbehandlungsraum (16) begrenzenden Anbauwänden (18) mittels Befestigungsvorrichtung (40) starr und luftdicht angeordnet ist. Im Fall, dass keine Anbauwände vorhanden sind, ist der Abdeckplanenrand (32) direkt auf dem Boden (50) luftdicht verankert.

Fig. 1A zeigt eine vergrößerte Darstellung des Abfallbehandlungsraumes (16a) aus Figur 1 im gehobenen Zustand. Der Abfallbehandlungsraum (16a) wird durch eine Abdeckplane (30) gebildet, die mit einer Hebe- und Senkvorrichtung (20) versehen ist. In einer Ausführungsform ist die Hebe- und Senkvorrichtung eine pneumatische Höhenverstellkonstruktion (22) und wird durch aufblasbare Schläuche (23) gebildet. Die Schläuche werden über einen Lüfter (24) mit Luft versorgt. Der Abdeckplanenrand (32) ist mittels einer Befestigungsvorrichtung (40) an der Außenseite (8) der Hallenwand (6) und auf den Anbauwänden (18) befestigt.

Fig. 1B zeigt eine vergrößerte Darstellung des Abfallbehandlungsraumes (16b) aus Figur 1 im gesenkten Zustand. Die Schläuche (23) der pneumatischen Hebe- und Senkvorrichtung (22) sind ohne Luft so dass die Abdeckplane (30) direkt auf der Abfallmiete liegt.

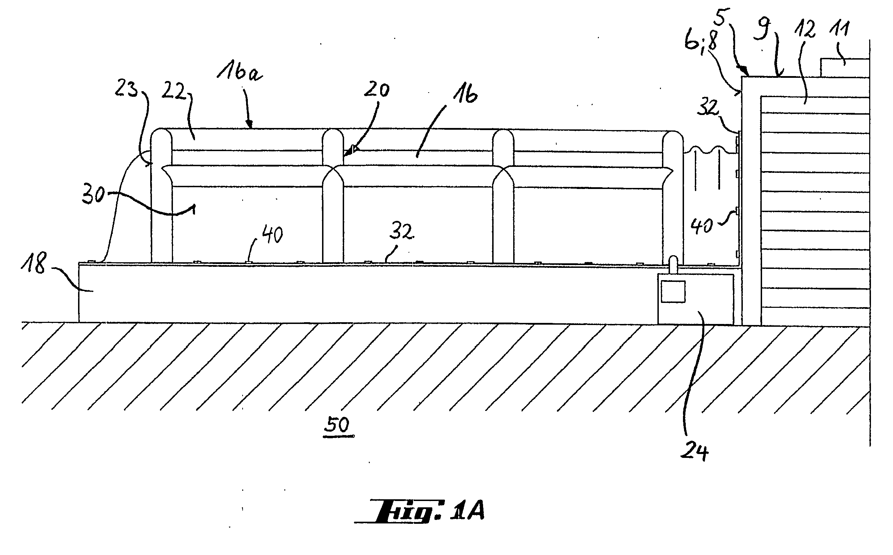

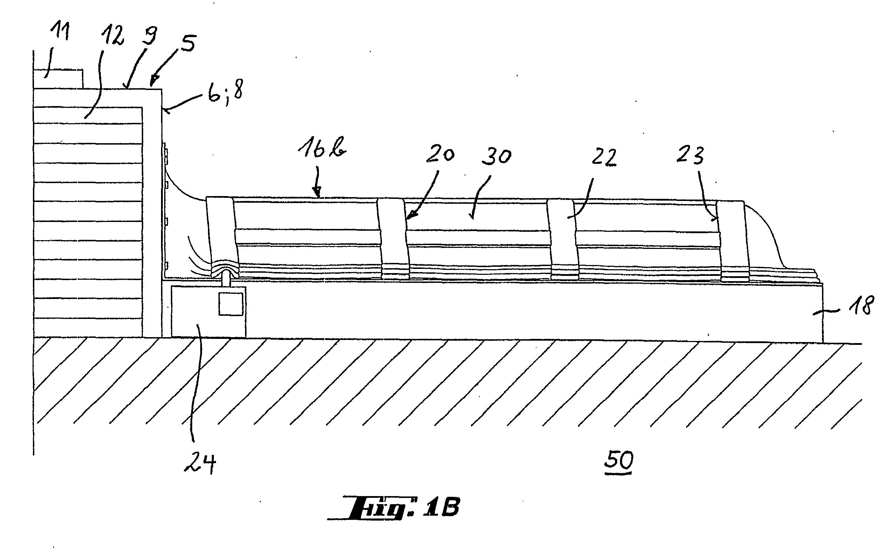

Fig. 2 zeigt eine Draufsicht auf die Abfallbehandlungsanlage (1) von Figur 1. An die Halle (5) ist ein Hallenlüfter (4) angeschlossen, der die mit Emissionen verunreinigte Luft aus dem Inneren der Halle (5) absaugt. Im Hallendach (9) ist eine Luftzufuhröffnung (11) angebracht, durch welche saubere Luft in die Halle (5) strömen kann. Die Luftzufuhröffnung (11) kann an beliebiger Stelle an der Halle vorgesehen sein. Auch das Öffnen des Hallenzugangs (12) sorgt für Frischluftzufuhr. In der Halle (5) wird ein ständiger Unterdruck erzeugt, das bedeutet, dass der Luftdruck in der Halle (5) kleiner als der Atmosphärendruck ist. Damit wird verhindert, dass verunreinigte Luft unkontrolliert in die Umgebung entweichen kann. Der Hallenlüfter (4) ist mit einem Abluftfilter (3), beispielsweise mit einem Biofilter verbunden, der die abgesaugte Hallenluft reinigt. Der Hallenlüfter (4) und der Abluftfilter (4) bilden zusammen eine Abgasreinigungseinrichtung.
An der jeweiligen Außenseite (8) der sich gegenüberliegenden seitlichen Hallenwänden (6) sind jeweils mehrere Abfallbehandlungsräume (16) rechenartig oder terminalförmig angeordnet. Jeder Abfallbehandlungraum (16) weist eine Hebe- und Senkvorrichtung (20) auf.

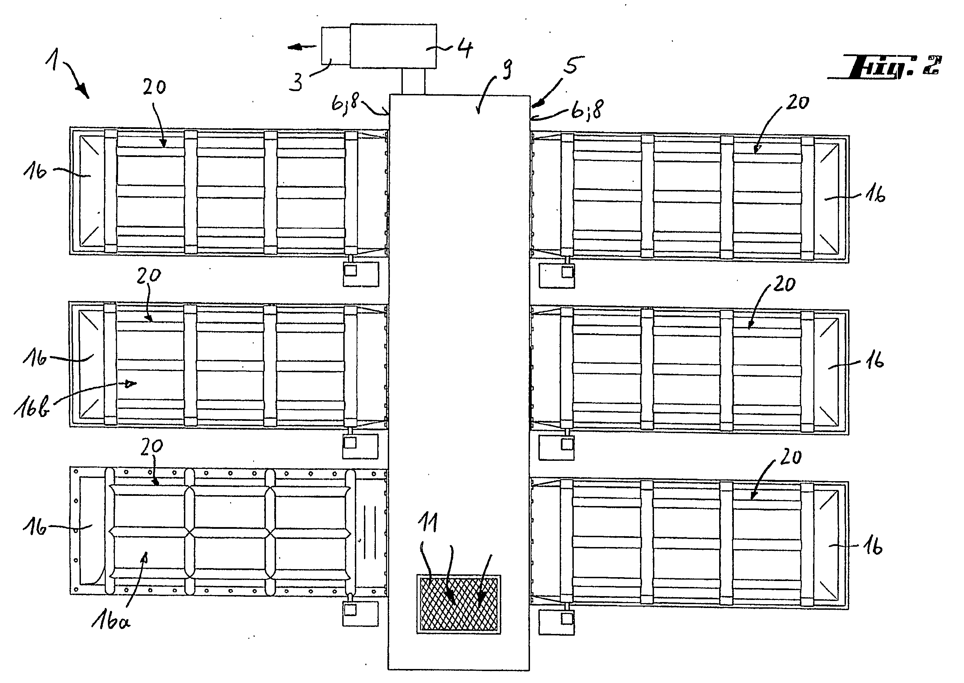

Figur 3 zeigt eine perspektivische Ansicht einer Ausführungsform der erfindungsgemäßen Abfallbehandlungsanlage (1) mit einer Halle (5) und jeweils drei angekoppelten Abfallbehandlungsräumen (16) an den sich gegenüberliegenden Seitenwänden (6). Die Halle (5) ist mit zumindest einer Hallenwand (6), einem Hallendach (9) und einem Hallenzugang (12) aufgebaut. Ein Hallenlüfter (4) ist an einer Stirnseite der Halle (5) angeordnet und saugt die Abluft aus der Halle. Je nach Größe der Anlage (1) können auch mehrere Lüfter und Biofilter vorgesehen sein. Im Hallendach (9) sorgt die Luftzufuhröffnung (ii) für frische Umgebungsluft in der Halle (5). An den Außenseiten (8) der seitlichen Hallenwänden (6) sind jeweils drei Abfallbehandlungsräume (16) angekoppelt. Jeder Abfallbehandlungsraum (16) wird durch eine Abdeckplane (30) mit einem Abdeckplanenrand (32) gebildet. Außerdem ist jeder Abfallbehandlungsraum (16) bodenseitig durch Anbauwände (18) begrenzt. Die Anbauwände (18) dienen als äußere seitliche Begrenzung des Abfallbehandlungsraumes (16), gleichzeitig wird der Transport des Abfalls in und aus dem Abfallbehandlungsraum (16) erleichtert und das Aufschichten des Abfalls zu einer Abfallmiete zwischen den Anbauwänden (18) vereinfacht. Der vordere Abfallbehandlungraum (16a) ist in gehobenem Zustand dargestellt, die übrigen Abfallbehandlungsräume (16b) sind in gesenktem Zustand dargestellt.
In der dargestellten Ausführungsform ist der Abdeckplanenrand (32) mittels einer Befestigungseinrichtung (40) an der Hallenwand (6) und an den Anbauwänden (18) befestigt. Die Abdeckplane (30) ist mit einer pneumatischen Hebe- und Senkvorrichtung (22) in Form von aufblasbaren Schläuchen (23) versehen. Jeweils zwischen jedem Abfallbehandlungsraum (16) und der Halle (5) ist eine Schleusenöffnung (nicht sichtbar) in der Hallenwand (6) vorgesehen, die das Innere der Halle (5) mit dem Inneren des Abfallbehandlungsraumes (16) verbindet. Die Schleusenöffnung ist überwiegend verschlossen und soll nur dann geöffnet werden, wenn Abfall (2) oder behandelter Abfall in oder aus dem Abfallbehandlungsraum (16) transportiert wird.
Jeder Abfallbehandlungsraum (16) kann aufgrund der Hebe- und Senkvorrichtung (20) der Abdeckplane (30) zwei Zustände einnehmen. Für den aeroben Behandlungsprozess ist der Abfallbehandlungsraum (16b) in einem gesenkten Zustand, das bedeutet, die Abdeckplane (30) liegt direkt auf dem Abfall. Lediglich zum Befüllen und Entleeren des Abfallbehandlungsraumes (16a) ist dieser in einem gehobenen Zustand. Die Hebe- und Senkvorrichtung (20) hebt sich und damit die mit der Vorrichtung verbundene Abdeckplane (30). Somit können Be - und Entladegeräte oder -fahrzeuge in das Innere des Abfalbehandlungsraumes (16a) gelangen. Der Behandlungsraum (16a) ist im gehobenen Zustand, das heißt, die Schläuche (23) sind aufgeblasen und haben die Abdeckplane (30) angehoben. Der Abfall kann durch die geöffnete Hallenwandöffnung in oder aus dem Abfallbehandlungsraum (16a) transportiert werden.
Die Abfallbehandlungsräume (16b) sind im gesenkten Zustand. Die Schläuche (23) sind ohne Luft, infolgedessen liegt die Abdeckplane (30) auf dem Abfall (2) und der aerobe Abbauprozess kann erfolgen. Die Hallenwandöffnung ist geschlossen.

Der Abdeckplanenrand (32) ist die äußere randseitige Begrenzung der Abdeckplane (30). Der Abfallbehandlungraum (16) ist über den Abdeckplanenrand (32) fest mit der Halle (5) als auch mit dem Boden (50) bzw. den Anbauwänden (18) verbunden. Dazu ist der Abdeckplanenrand (32) zur Hallenwand (6) und zum Boden bzw. den Anbauwänden mittels einer Befestigungsvorrichtung (40) starr und luftdicht angeordnet. Damit wird ein zur Umgebung geschlossener Abfallbehandlungsraum (16) geschaffen. Die luftdichte Befestigung des Abdeckplanenrandes (32) bewirkt, dass keine Fehlluftströme an Abluft aus dem Inneren des Abfallbehandlungsraumes (16) in die Umgebung gelangen können, beispielsweise über Luftbrücken zwischen dem Abdeckplanenrand (32) und der Hallenwand (6). Die starre Befestigung stellt eine unbewegliche Befestigung in der Hinsicht dar, dass der Abdeckplanenrand (32) auch während der Hebe- und Senkvorgänge der Abdeckplane (30) stets fest und unbeweglich in seiner befestigten Position verbleibt, sodass der Abfallbehandlungsraum (16) immer als ein geschlossenes System vorliegt.
Der Abfallbehandlungsraum (16) hat einen Vorder- und einen Endbereich und dazwischen liegende Seitenbereiche. Der Vorderbereich grenzt an die Hallenwand (6) und umgibt die Hallenwandöffnung. Der Abdeckplanenrand (32) im Vorderbereich ist an der Hallenwand (6) befestigt. Der Abdeckplanenrand (32) der Seitenbereiche und im Endbereich ist auf oder an den Anbauwänden (18) befestigt. In einer weiteren Ausführungsform ist der Abdeckplanenrand (32) der Seitenbereiche und im Endbereich direkt auf dem Boden (50) befestigt.

Die Hebe- und Senkvorrichtung (20) umfaßt eine pneumatische Stützkonstruktion (22) oder eine mechanische Höhenverstellkonstruktion (26).
Die pneumatische Stützkonstruktion (22) weist eine Anzahl von mit mindestens einem Fluid füllbaren und mindestens teilweise miteinander verbundener Stützelemente (23) auf. Das die Stützelemente füllende Fluid kann ein Gas, ein Dampf, eine Flüssigkeit oder ein Gas und eine Flüssigkeit sein. Das bedeutet, dass die Stützelemente entweder mit mindestens einem Gas oder mit mindestens einer Flüssigkeit oder mit mindestens einem Gas und mindestens einer Flüssigkeit gefüllt werden können. Die Stützelemente sind mindestens gasdicht ausgestaltet. Vorzugsweise sind die Stützelemente aufblasbare Schläuche (23).
In der Ausführung in Figur 3 bildet die Hebe- und Senkvorrichtung (20) einen halbrundförmigen Tunnel mit einem Vorderbereich und einem Endbereich, wobei der Vorderbereich über den Abdeckplanenrand (32) an der Hallenwand (6) befestigt ist. Je nach Ausgestaltung der Stützelemente kann die Vorrichtung im aufgestellten Zustand jede beliebige Form annehmen wie beispielsweise quaderförmige, kuppel-, kegel-, oder pyramidenförmige Ausgestaltungen.
Die pneumatische Hebe- und Senkvorrichtung (22) kann beliebig viele Stützelemente aufweisen. Mindestens sind jedoch zwei Stützelemente erforderlich, um eine ausreichende Befestigung der Abdeckplane (30) zu ermöglichen und um der gesamten Vorrichtung die notwendige Stabilität zu geben. In Figur 3 liegen vorzugsweise vertikal und horizontal angeordnete Stützelemente vor, die kreuzweise zueinander angeordnet sind. Die Stützelemente können auch schräg in einem bestimmten Winkel zum Erdboden verlaufen.

Jede pneumatische Hebe- und Senkvorrichtung (22) weist mindestens ein Fluideinlaß (51) und mindesten ein Fluidauslaß (53) auf. In einer Ausführungsform ist der Fluideinlaß (51) gleichzeitig auch der Fluidauslaß (53). Jedes Stützelement kann einen eigenen Fluideinlaß (51) und Fluidauslaß (53) aufweisen oder, wenn die Stützelemente so miteinander verbunden sind, dass das Fluid durch alle Stützelemente fließen kann, reicht ein Fluideinlaß (51) und ein Fluidauslaß (53) für alle Stützelemente aus. Der Fluideinlaß (51) ist eine Öffnung in einem Stützelement, in welche ein Anschlußstück zum Lüfter (24) ein- oder angeschweißt ist. Das Anschlußstück kann beispielsweise ein PVC-Rohr sein. Der Fluidauslaß (52) ist ein handelsübliches Ventil beispielsweise der Firma Scoprega S.p.A., Mailand, Italien. Ein Lüfter (24) ist außerhalb der pneumatischen Hebe- und Senkvorrichtung (23) an den Fluideinlaß (51) angeschlossen und versorgt die Stützelemente mit mindestens einem Fluid.

Die Stützelemente (23) sind mindestens teilweise miteinander verbunden, das umfaßt alle Ausführungsformen, in denen die Stützelemente (23) unmittelbar miteinander an ihren Kontaktpunkten verbunden sind, die Stützelemente (23) nur an einzelnen Kontaktpunkten miteinander verbunden sind, nur einzelne Stützelemente miteinander verbunden sind oder die Stützelemente (23) mittelbar über Hilfsmittel wie beispielsweise Verbindungsleisten, -schienen oder -schnüren miteinander verbunden sind. Die Stützelemente (23) können beispielsweise nur über die Abdeckplane miteinander verbunden sein. Vorzugsweise ist jedes Stützelement (23) mit einem benachbarten Stützelement (23) an einzelnen Kontaktpunkten verbunden. Die Verbindung der Stützelemente (23) bewirkt, dass im aufgestellten Zustand eine stabile und sich selbst stützende Konstruktion entsteht. In einer Ausführungsform liegen die Stützelemente (23) an ihren Kreuzungs- oder Kontaktpunkten übereinander und sind derart miteinander verbunden, dass die sich berührenden Oberflächen der horizontalen und vertikalen Stützelemente (23) miteinander verklebt, vernäht, verschweißt oder auf andere Art miteinander verbunden sind.
In einer anderen Ausführungsform sind die Stützelemente (23) an den Kreuzungs- oder Kontaktpunkten so miteinander verbunden, dass die Querschnitte der horizontalen Stützelemente in die Querschnitte der vertikalen Stützelemente hineinragen. Damit kann das mindestens eine Fluid von einem Punkt aus durch die gesamte Konstruktion der Stützelemente (23) strömen. Das ist besonders vorteilhaft, weil lediglich ein Lüfter (24) an die Hebe- und Senkvorrichtung angeschlossen werden muß. Außerdem ist diese Struktur besonders stabil, da sich eine vorteilhafte Druckverteilung einstellt.
In einer weiteren Ausführungsform sind lediglich einzelne, vertikal aufgestellte Stützelemente vorgesehen, welche über ein horizontales Fluidrohr miteinander verbunden sind. Das Fluidrohr kann im Dachgiebel oder in Bodennähe verlaufen und verteilt das über den Fluideinlaß (51) einströmende Fluid auf alle Stützelemente. Das Fluidrohr ist bevorzugt ein starres Kunststoff- oder Metallrohr, welches dem am Fluideinlaß (51) anliegenden Druck standhalten muß. Es kann aber auch aus flexiblen gasdichten Materialien gefertigt sein.

Letztendlich können die Stützelemente (23) in jeder beliebigen Anordnung zueinander angeordnet sein soweit damit im aufgestellten Zustand ein dreidimensionales Gebilde geschaffen wird.

Die Stützelemente (23) sind mit mindestens einem Fluid wie Flüssigkeiten oder Gasen befüllbar, d.h. sie müssen einen für Gase oder Flüssigkeiten durchströmbaren Querschnitt aufweisen. Eventuelle Verstärkungselemente im Inneren der Stützelemente (23) dürfen deren Durchströmbarkeit nicht wesentlich behindern. Für eine ausreichende Stabilität und Auflagefläche für die Abdeckplane (30) sollte der Querschnitt der Stützelemente (23) einen Durchmesser von mindestens 10cm aufweisen. Vorzugsweise beträgt der Durchmesser 50cm. In einer weiteren bevorzugten Ausführungsform haben die Stützelemente einen Durchmesser von mindestens 80cm, vorzugsweise liegt der Durchmesser zwischen 90cm und 110cm.
Die Stützelemente können beliebige dreidimensionale Gebilde sein, wie z.B. Schläuche oder andere Hohlkörper. Die Stützelemente (23) sind in einer bevorzugten Ausführungsform Schläuche. Die Stützelemente (23) können jede beliebige Querschnittsform haben, wobei ein runder Querschnitt besonders bevorzugt ist. Ein runder Querschnitt ist einfach herzustellen und erlaubt eine optimale Druckverteilung innerhalb der Stützelemente (23). Die Stützelemente können beispielsweise auch einen ovalen Querschnitt aufweisen.
In einer Ausführungsform sind die Stützelemente mit Gas aufblasbare Schläuche (23). Als Gas wird vorzugsweise Luft verwendet, die einen Überdruck von mindestens 200Pa in den Stützelementen (23) aufweist. Vorzugsweise hat die Luft einen Überdruck von mindestens 10kPa. In einer Ausführungsform wird mit einem Überdruck zwischen 15 und 22kPa gearbeitet. Neben Luft kann auch Helium oder andere verfügbare Gase zur Anwendung kommen.
In einer weiteren Ausführungsform sind die Stützelemente (23) mit einer Flüssigkeit wie beispielsweise Wasser füllbar.

Die Stützelemente (23) können auch mit einer Flüssigkeit und einem Gas gefüllt sein, wobei dann vorzugsweise die Flüssigkeit in einem unteren Teil der Stützelemente (23) und das Gas in einen oberen Teil der Stützelemente (23) eingebracht wird. Dabei umfaßt der untere Teil den bodennahen Bereich der Stützelemente (23) wie beispielsweise den Wandbereich und der obere Teil umfaßt einen bodenfernen Bereich wie beispielsweise den Dachbereich. Das hat den Vorteil, dass die Flüssigkeit gleichzeitig die Vorrichtung am Boden (50) stabilisiert.

Um einen zu hohen Druck in den Stützelementen zu verhindern, ist mindestens ein Überdruckventil (55) vorgesehen. Dieses mindestens eine Überdruckventil (55) öffnet sich beispielsweise bei einem Innendruck von mehr als 25kPa und verhindert somit eine eventuelle Zerstörung der Stützelemente durch Überdruck. Beispielsweise kann ein Überdruckventil des Herstellers Halkey Roberts, St Petersburg, Florida, USA zur Anwendung kommen.

Als Material für die Stützelemente (23) dient ein wasserdichtes und luftdichtes Material, wie beispielsweise ein PVC beschichtetes Trägergewebe. Das Material sollte ausreichend witterungsbeständig und strapazierbar sein, um eine hohe Lebensdauer zu ermöglichen. Vorzugsweise ist das Material flexibel und damit faltbar bzw. drapierbar, damit im abgesenkten Zustand die Stützelemente (23) in sich zusammenfallen können. Das Merkmal der Drapierbarkeit ist für die vorliegende Erfindung wichtig, damit während und nach dem Entleeren der Stützelemente (23) das Zusammenfallen der Vorrichtung kontrollierbar ist, um ein genaues Plazieren der Abdeckplane (30) auf der Oberfläche der Abfallmiete zu erreichen.

Die Abdeckplane (30) des Abfallbehandlungsraumes (16) ist entweder auf der Oberseite oder auf der Unterseite der Stützelemente (23) einer pneumatische Hebe- und Senkvorrichtung (20) angeordnet.
In einer weiteren Ausführungsform ist die Abdeckplane (30) zwischen den Stützelementen (23) angeordnet und zwar so, dass die von den Stützelementen (23) in Umfangsrichtung begrenzten Flächen von der Abdeckplane (30) ausgefüllt sind.
Vorzugsweise befindet sich die Abdeckplane (30) auf der Unterseite der Stützelemente. Die Abdeckplane (30) kann mit jeder bekannten Befestigungsart an den Stützelementen (23) befestigt werden, dazu gehören Befestigungsmöglichkeiten wie Binden, Nähen, Kleben, Verschweißen, mit Druck- oder Magnetknöpfen, mit Klettverschlüssen, mit Haken oder Reißverschluß. Vorzugsweise ist die Abdeckplane (30) lösbar an den Stützelementen (23) befestigt, um ein leichtes und schnelles Wechseln der Abdeckplane (30) bei Verschmutzung oder Beschädigung dieser zu ermöglichen.

In einer Ausführungsform bedeckt die Abdeckplane (30) die gesamte Unterseite der Stützelemente (23).

In einer anderen Ausführungsform bedeckt die Abdeckplane (30) lediglich die Unterseite des Dachbereiches der Hebe- und Senkvorrichtung (20). Diese besonders kostengünstige Ausführung verwendet zusätzlich eine wasserdichte Schutzschicht (56), die im Wandbereich der Hebe- und Senkvorrichtung (20) angebracht ist. Diese wasserdichte Schutzschicht (56) ist in der Regel kostengünstiger als die Abdeckplane (30) und weist ein robustes abrasionsbeständiges Material wie beispielsweise ein PVC beschichtetes Trägergewebe auf. Damit wird ein zusätzlicher Schutz des Abfallbehandlungsraumes (16) gegen Beschädigungen der Seitenwände im gehobenen Zustand durch Maschinen und Fahrzeugen erreicht.

Für eine ausreichende Belüftung der Abfallmiete in dem Abfallbehandlungsraum hat die Abdeckplane (30) eine ausreichende Luftdurchlässigkeit aufzuweisen, um die aeroben Abbauprozesse der organischen Bestandteile im Abfall zu gewährleisten. Vorzugsweise liegt die Luftdurchlässigkeit der Abdeckplane (30) zwischen 3 und 100 m³/m²/h bei einer angelegten Druckdifferenz von 200Pa.
Die Abdeckplane (30) ist flüssigkeitsdicht bei einen Wassereintrittsdruck von größer 10kPa, vorzugsweise größer 50kPa, wobei der Wassereintrittsdruck bis zu einer Höhe von 1MPa gehen kann.

Der Wasserdampfdurchgangswiderstand Ret der Abdeckplane (30) beträgt weniger als 15m²Pa/W, vorzugsweise weniger als 10m²Pa/W.
Die Abdeckplane ist ein gasdurchlässiges und wasserdichtes Textil, eine gasdurchlässige und wasserdichte Membrane oder ein Laminat mit einer gasdurchlässigen und wasserdichten Membrane. Als Textil kann ein dichtverpresstes oder dichtverwebtes Textil, wie beispielsweise ein hochfestes Polyestergewebe, verwendet werden.
Die Abdeckplane (30) muß aus einem flexiblen und somit faltbaren und drapierbaren Material sein, damit es im gesenkten Zustand der Hebe- und Senkvorrichtung (20) auf die Oberfläche der Abfallmiete plaziert sowie überschüssiges Material an Abdeckplane und Stützelementen um die Abfallmiete herum gefaltet werden kann.

Vorzugsweise wird als flüssigkeitsdichte und gasdurchlässige Abdeckplane ein Laminat mit einer Membrane (34) und mindestens einer textilen Schicht (36) verwendet. Vorzugsweise ist die Membrane (34) porös, die Poren der Membrane müssen ausreichend groß sein, um den notwendigen Gasdurchsatz zu ermöglichen. Bei der Membrane (34) handelt es sich beispielsweise um ein Material aus der Gruppe der Polyolefine, Polyester, Polyvinylchloride, Polyvinylidenchloride, Polyurethane oder Fluorpolymere. Vorzugsweise ist die poröse Membrane eine mikroporöse Membrane. Membranen sind dünn, leicht, flexibel und drapierfähig. Zusätzlich sind sie wasserdampfdurchlässig, luftdurchlässig und wasserdicht.

Bevorzugte mikroporöse Membranen beinhalten Fluorpolymere wie beispielsweise Polytetrafluorethylen; Polyolefine wie Polyethylen oder Polypropylen; Polyamide, Polyester; Polysulfone, Polyethersulfone und Kombinationen davon; Polycarbonate; Polyurethane. Vorzugsweise wird eine Membrane aus gerecktem Polytetrafluorethylen (ePTFE) verwendet. Die Membrane aus ePTFE liegt mit einer Dicke zwischen 5-500µm, vorzugsweise zwischen 15-60µm vor.
Dieses Material zeichnet sich durch eine Vielzahl von offenen, miteinander verbundenen Hohlräumen aus, einem großem Hohlraumvolumen und einer großen Stärke. Expandiertes Polytetrafluorethylen (ePTFE) ist weich, flexibel, hat stabile chemische Eigenschaften, eine hohe Durchlässigkeit gegenüber Gasen sowie Dämpfen und eine Oberfläche mit einer guten Abweisung gegen Verunreinigungen. Aufgrund seiner hohen Kälteknickbeständigkeit ist es für den Einsatz in allen Klimazonen geeignet.
Weiterhin ist dieses Material durchlässig für Gas. Die Porosität und die Porengröße ist so gewählt, dass die Gasdiffusion nicht behindert wird. Die durchschnittliche Porengröße kann 0,1-100µm betragen, vorzugsweise 0,2 - 10µm, bestimmt nach dem oben beschriebenen Coulter-Test. Die Porosität beträgt 30 - 90%, vorzugsweise 50 - 80%. Gleichzeitig ist das Material wasserdicht. Ein Verfahren zur Herstellung solcher poröser Membranen aus gerecktem PTFE ist beispielsweise in den Patenten US 3,953,566 und US 4,187,390 offenbart.

Vorzugsweise ist die mikroporöse Membrane mit einem textilen Trägermaterial versehen, welches der Membrane einen zusätzlichen Schutz und Festigkeit verleiht. Das Trägermaterial kann mit einer kontinuierlichen oder diskontinuierlichen Klebstoffschicht auf mindestens einer der Oberflächen der Membrane auflaminiert sein. Vorteilhafterweise ist das Trägermaterial ein textiles Flächengebilde aus gewebten, gewirkten oder gestrickten, natürlichen oder synthetischen textilen Materialien. Auch Gelege und Vliese können verwendet werden. Als textile Materialien sind besonders Polyester, Polyamide, Polyethylen, Polyacrylate, Polypropylen, Glasfaser, Fluorpolymer oder ein aus PTFE gewebtes Textil geeignet. Es ist vorgesehen, dass das Trägermaterial nach außen zur Atmosphäre hin angeordnet ist. Alternativ kann auf der anderen Membranoberfläche ein weiteres textiles Flächengebilde angeordnet sein.

In einer weiteren Ausführungsform ist die Membrane oleophobiert. Eine Oleophobierung der Membrane erfolgt derart, dass die Porosität der Membrane nicht deutlich verringert wird. Vorzugsweise hat die Membrane eine Ölrate von > 1, idealerweise ist die Ölrate > 5, so dass das Befeuchten und Verschmutzen mit organischen Substanzen dauerhaft vermieden wird. Das Oleophobieren wird beispielsweise in der DE 43083692 beschrieben. Besonders bevorzugt wird für die vorliegende Erfindung eine oleophobierte ePTFE Membrane. Die oleophobierte mikroporöse Membrane kann mindestens eine textile auflaminierte Trägerschicht aufweisen.
Bei den textilen Trägermaterialien werden vergleichbar hohe Ölraten durch Verwendung kommerziell verfügbarer Fluorkarbon-Beschichtungen) erreicht. Gewöhnlich wird ein Oleophobiermittel in flüssiger Form auf das zu oleophobierende Material aufgebracht, wie beispielsweise durch Tauchen, Tränken, Sprühen, Beschichten, Streichen, Walzen.

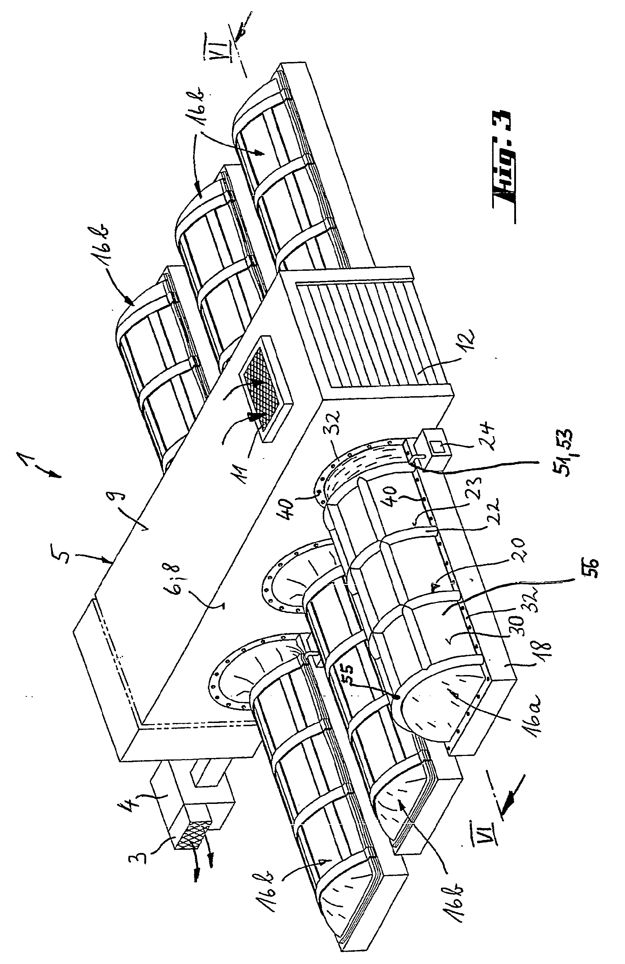

Eine besonders bevorzugte Abdeckplane in Form eines 3-Lagen-Laminates ist in Figur 4 dargestellt. Zwischen zwei textilen Trägermaterialien (36) ist eine wasserdichte, wasserdampfdurchlässige Membrane (34) angeordnet. Die Membrane ist eine mikroporöse Membrane, vorzugsweise aus ePTFE. Die Porengröße der Membrane beträgt 0,1 bis 100µm, vorzugsweise 0,2 bis 10µm. Eine solch geringe Porengröße verhindert, dass Keime und Bio-Aerosole nach außen dringen können. Gleichzeitig ist für einen ausreichenden Gasaustausch mit der Umgebung gesorgt.
Ein solches Laminat wird beispielsweise in der WO 01/21394 A1 beschrieben und ist über die Firma W.L Gore & Associates GmbH, Putzbrunn bei München, unter dem Namen Gore®-Cover erhältlich.

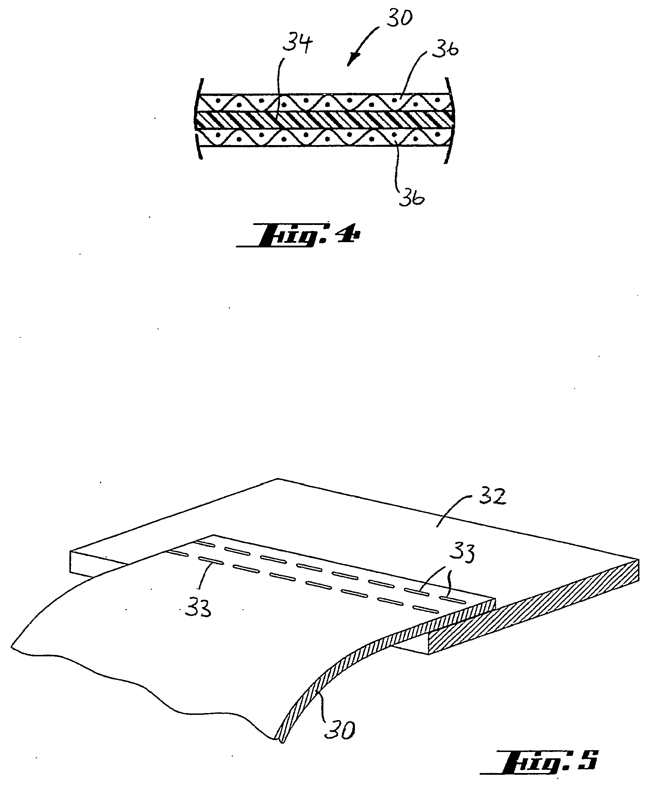

Figur 5 zeigt eine Ausgestaltung des Abdeckplanenrandes (32). Dazu wird an den äußeren Rand der Abdeckplane (30) ein flächiger Verstärkungsstreifen befestigt. Dieser Verstärkungsstreifen bildet den Abdeckplanenrand (32) und wird beispielsweise durch einen flächigen Streifen durch beidseitig mit PVC beschichteten Polyestergewebe gebildet. Der Verstärkungsstreifen ist aufgrund seiner robusten Materialstruktur sehr widerstandsfähig gegenüber der Befestigungsvorrichtung. Dieser Streifen ist entlang des äußeren Randes der Abdeckplane (30) beispielsweise mit mehreren Nähten (33) befestigt, um unter anderem zu verhindern, dass der Verstärkungsstreifen durch die Beanspruchung während der Hebe- und Senkvorgänge von der Abdeckplane (30) gerissen wird. Anstelle von Nähten können auch Verfahren wie Kleben oder Schweißen zur Anbringung des Streifens angewandt werden.

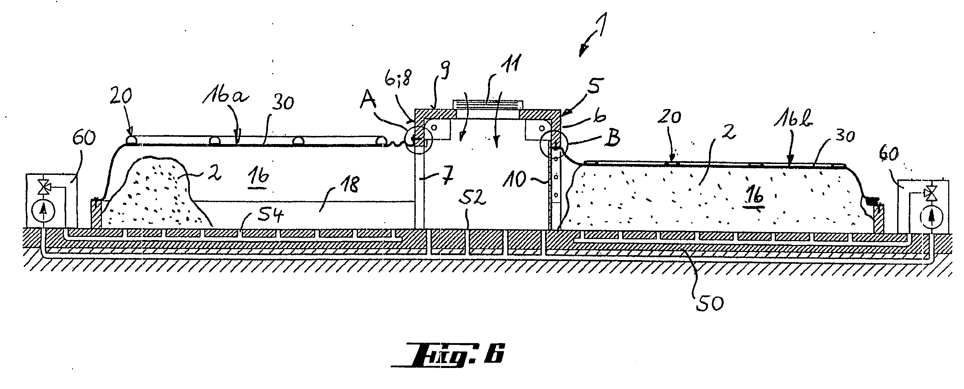

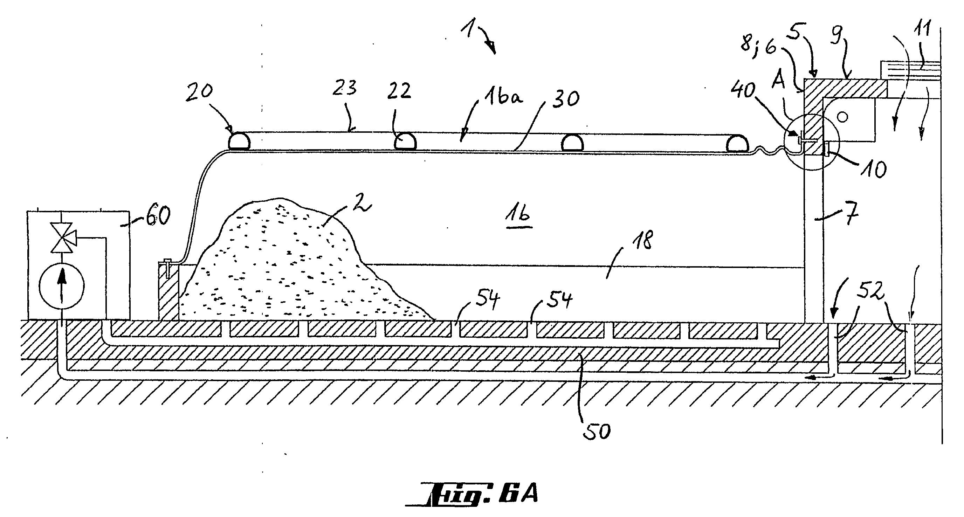

Figur 6 zeigt einen Schnitt gemäß der Schnittlinie VI-VI durch die Abfallbehandlungsanlage (1) gemäß Figur 3. Zwischen den Abfallbehandlungsräumen (16a) und (16b) liegt die Halle (5). Im Dach der Halle (5) ist eine Luftöffnung (11) für die Zufuhr von frischer Luft in das Innere der Halle. Die Abfallbehandlungsräume (16a) und (16b) sind jeweils über eine verschließbare Hallenwandöffnung (7) mit der Halle (5) verbunden. Die Hallenwandöffnung (7) ist beispielsweise mittels einer Tür (10) in Form eines Rolltors verschließbar. Der Abfallbehandlungsraum (16a) ist im gehobenen Zustand dargestellt. Die Abdeckplane (30) ist mittels der Hebe- und Senkvorrichtung (20) angehoben und befindet sich in einem Abstand über der Abfallmiete (2). Die Hallenwandöffnung (7) ist geöffnet, so dass beispielsweise Radlader hineinfahren können zum Auf- oder Abbau der Abfallmiete. Der Abfallbehandlungsraum (16b) befindet sich im gesenkten Zustand, somit ist die Hebe- und Senkvorrichtung (20) gesenkt und die Abdeckplane (30) bedeckt unmittelbar die Abfallmiete. Die Hallenwandöffnung (7) ist geschlossen.
Der jeweilige Abdeckplanenrand (32) jedes Behandlungsraumes (16a, 16b) ist mit einer Befestigungsvorrichtung (40) an der Hallenwand (6) und an den Anbauwänden (18) befestigt. Die Befestigungsvorrichtung (40) dient dazu, den Abdeckplanenrand (32) zur Hallenwand (6) und zum Boden (50) starr und luftdicht anzuordnen. Die Befestigungsvorrichtung (40) umfaßt Fixierelemente wie beispielsweise Schrauben, Nägel, Stifte, Klemmen, Nieten, welche durch den Abdeckplanenrand (32) hindurch in der Hallenwand (6) oder den Anbauwänden (18) bzw. dem Boden (50) flächig und luftdicht verankert sind. Somit verbleibt der Abdeckplanenrand (32) auch während der Hebe- und Senkvorgänge starr und unbeweglich in seiner befestigten Position und der gesamte Abfallbehandlungsraum (16) bleibt somit geschlossen zur Umgebung. Die Anordnung des Abdeckplanenrandes (32) mittels der Befestigungsvorrichtung (40) ist außerdem luftdicht, das heißt, dass keine Fehlluft und damit keine schädlichen Emissionen aus dem Inneren des Abfallbehandlungsraumes (16) entweichen können.
Somit ist der gesamte Abfallbehandlungsraum partikeldicht was bedeutet, dass keine Partikel >0.3µm den Abfallbehandlungsraum verlassen können, weder durch die Abdeckplane noch durch die Verbindung zwischen Abdeckplane und Hallenwand bzw. Anbauwänden. Lediglich beim Öffnen der Hallenwandöffnung (7) können schädliche Emissionen in das Halleninnere entweichen, dort werden diese jedoch abgesaugt und einer Abluftbehandlung zugeführt.
Aufgrund von Unebenheiten in der Oberfläche der Wände und des Bodens ais auch aufgrund von Faltenbildung des Abdeckplanenrandes (32) beim Anordnen an den Wänden oder dem Boden kann es erforderlich sein, mindestens eine Dichtlage (42) aus einem anpassungsfähiges Material zwischen Abdeckplanenrand (32) und Wand bzw. Boden anzuordnen. Die Dichtlage dient dazu, die Unebenheiten in den Wänden oder Boden auszugleichen und zu beseitigen, um eine luftdichte Befestigung des Abfallbehandlungsraumes (16) an der Halle herbeizuführen. Mit Hilfe der Dichtlage oder des anpassungsfähigen Materials (42) soll ein luftdichter Formschluß zwischen den Wänden (6, 18) und dem Abdeckplanenrand (32) gebildet werden. Damit wird verhindert, dass sich Luftbrücken innerhalb der Befestigung des Abdeckplanenrandes (32) ausbilden und Fehlluft aus dem Inneren des Abfallbehandlungsraumes in die Umgebung gelangen kann. In einer Ausführungsform ist das anpassungsfähige Material ein elastisches und weiches Material wie beispielsweise ein Gummi oder Schaumstoff. Als anpassungsfähiges Material kann ein flexibles Material aus der Materialgruppe enthaltend Schaumstoff, Gummi, beschichtete Trägerstoffe, Polymerstreifen, beschichtete Textilien gewählt werden. Ausgestaltungen der Befestigungsvorrichtung (40) sind in den Figuren 7 bis 9 dargestellt.

In einer weiteren Ausführungsform sind im Boden der Halle (5) eine Anzahl von ersten Bodenöffnungen (52) vorgesehen. Zusätzlich zu dem Hallenentlüfter (4) kann Luft aus der Halle (5) durch erste Bodenöffnungen (52) abgeführt werden.
Im Boden des Inneren des Abfallbehandlungsraumes (16a, 16b) sind eine Anzahl von zweiten Bodenöffnungen (54) vorgesehen. Diese dienen dazu, in den Abfallbehandlungsraum Luft über separate Lüfter (60) zu leiten, damit die Abfallmiete belüftet wird.

In einer weiteren Ausführungsform wird die Abluft über die ersten Bodenöffnungen (52) aus der Halle (5) gesaugt und über Bodenkanäle den zweiten Bodenöffnungen (54) zugeführt. Grundsätzlich sind jedem einzelnen Abfallbehandlungsraum einzelne Lüfter (60) zugeordnet, die während des aeroben Behandlungsprozesses Luft oder bei Bedarf Abluft in das Innere des Abfallbehandlungsraumes (16) leiten. Somit kann die abgesaugt Hallenluft zur Belüftung des Abfalls verwendet werden. Diese Luft gelangt dann durch die gasdurchlässige Membrane (34) der Abdeckplane (30) wieder in die Umgebung. Eine Reinigung der Luft findet dann in der Porenstruktur der Membrane statt.

Fig. 6A zeigt einer vergrößerte Darstellung des Abfallbehandlungsraumes (16a) aus Figur 6 im gehobenen Zustand. Die pneumatische Hebe- und Senkvorrichtung (22) ist mit Luft gefüllt und die Hallenwandöffnung 7 ist zum Zwecke des Befüllens oder Entleerens des Abfallbehandlungsraumes (16a) geöffnet. Zweite Bodenöffnungen (54) dienen der Zufuhr von Belüftungsluft. Die verunreinigte Luft aus der Halle (5) wird mit dem Hallenlüfter (4) über die ersten Bodenöffnungen (52) aus dem Inneren der Halle (5) abgesaugt und nach dem Reinigen in einem Abluftfilter (3) an die Umgebung abgegeben.

Fig. 6B zeigt eine vergrößerte Darstellung des Abfallbehandlungsraumes (16b) aus Figur 6 im gesenkten Zustand. Die Schläuche (23) der pneumatische Hebe- und Senkvorrichtung (22) sind ohne Luft, deshalb liegt die Abdeckplane (30) direkt auf dem Abfall (2). Die Hallenwandöffnung (7) ist mittels Tür (10) verschlossen. Die abgesaugt Abbluft aus dem Inneren der Halle (5) wird als Belüftungsluft über die zweiten Bodenöffnungen (54) in den Abfallbehandlungsraum (16b) geleitet.

Figur 7 zeigt eine vergrößerte Darstellung einer Ausgestaltung der Befestigungsvorrichtung (40) in dem Bereich A in Figur 6. Zwischen Hallenwand (6) und dem Abdeckplanenrand (32) ist eine Dichtlage aus einem anpassungsfähiges Material (42) angeordnet. Dieses Material dient dazu, Unebenheiten in der Hallenwand (6) auszugleichen, damit sich keine Luftkanäle zwischen Abdeckplanenrand (32) und Hallenwand (6) bilden. Die Dichtlage ist in einer Ausführungsform eine dauerhaft verformbare PVC Folie und liegt als flächiger Streifen mit einer Breite von beispielsweise 10cm vor. Auf dem Abdeckplanenrand (32) ist eine Platte (46) angeordnet, die zusammen mit den anderen Lagen an der Hallenwand (6) verschraubt wird, um einen flächigen Anpressdruck auf den Abdeckplanenrand (32) und die Dichtlage (42) zu gewährleisten. Diese Platte (46) kann eine Metallplatte mit regelmäßig angeordneten Öffnungen für die Befestigungsschrauben (44) sein. Die Schrauben (44) werden durch die Platte (46), dem Abdeckplanenrand (32) und der Dichtlage (42) in der Hallenwand (32) fixiert. Mit Anziehen der Schrauben drückt die Metallplatte flächig auf den Abdeckplanenrand (32) und die Dichtlage (42) und presst die Lagen starr gegen die Hallenwand (6). Es bildet sich ein luftdichter Formschluß zwischen Hallenwand (6) und Abdeckplanenrand (32). Im Bereich der Anbauwände (18) wird der Abdeckplanenrand (32) nach dem gleichen oben beschriebenen Befestigungsaufbau luftdicht und starr auf oder an der jeweiligen Anbauwand befestigt. Das gleiche gilt bei einer Befestigung auf dem Boden (50).

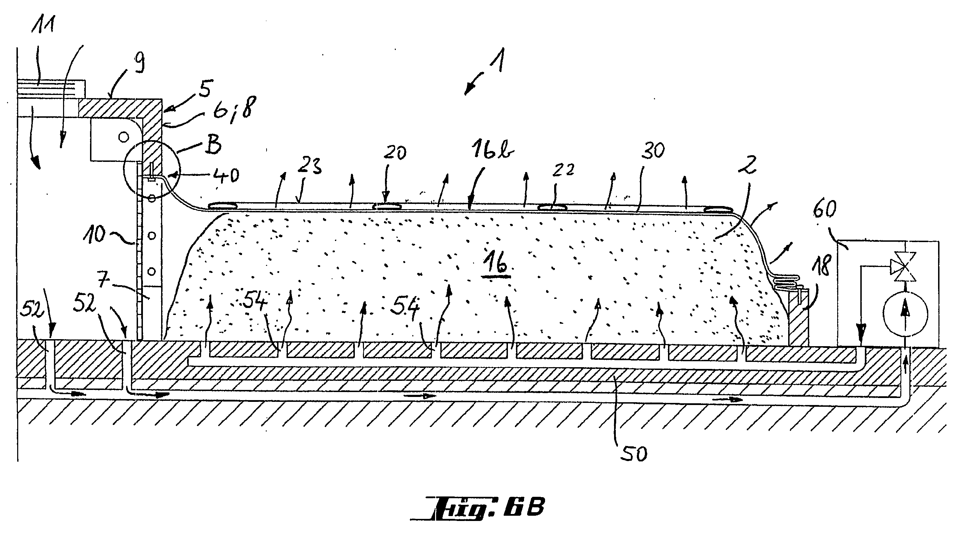

Figur 8 zeigt eine vergrößerte Darstellung einer weiteren Ausgestaltung der Befestigungsvorrichtung (40) in dem Bereich A in Figur 6. Hier ist der Abdeckplanenrand (32) durch einen Verstärkungsstreifen gemäß Figur 5 gebildet. Der Verstärkungsstreifen weist eine ausreichende Festigkeit auf, so dass auf die Platte (46) verzichtet werden kann. Die Schrauben (44) fixieren somit den Vertärkungsstreifen (32) und die Dichtlage (42) luftdicht an der Hallenwand (6).

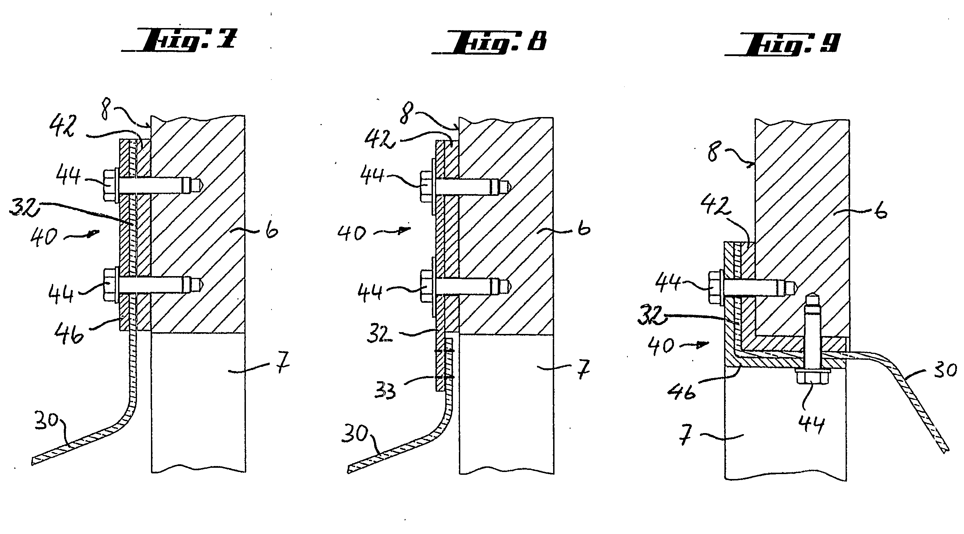

Figur 9 zeigt eine vergrößerte Darstellung einer weiteren Ausgestaltung der Befestigungsvorrichtung (40) in dem Bereich B in Figur 6. Die Anordnung entspricht weitestgehend Figur 7, allerdings sind die Lagen winklig an der Hallenwandöffnung (7) und an der Innenseite der Hallenwand (6) befestigt.

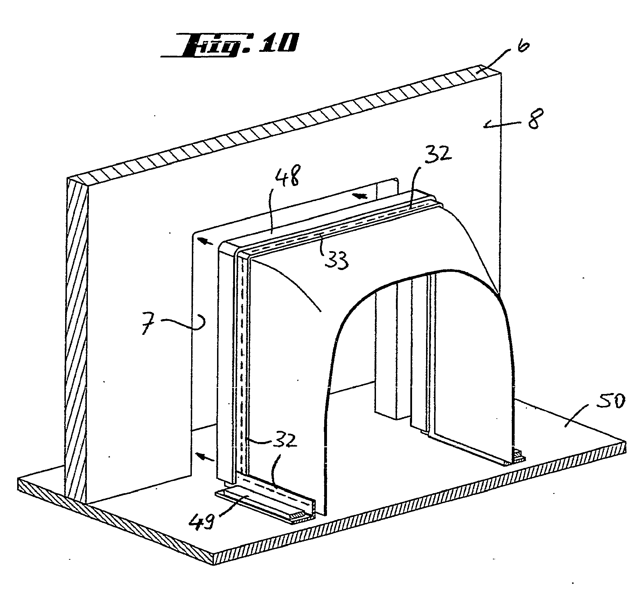

Figur 10 zeigt eine weitere Ausgestaltung der Befestigungsvorrichtung (40). In einem Rahmen (48) entsprechend der Form der Hallenwandöffnung (7) wird der Abdeckplanenrand (32) luftdicht geklemmt. Der Rahmen (48) wird luftdicht in die Hallenwandöffnung (7) eingepaßt. Mit dieser Ausgestaltung wird eine besonders einfache Art der Befestigung der Abdeckplane (30) an der Hallenwand (6) bewirkt. Der verbleibende Abdeckplanenrand kann mittels eines horizontal geformten Klemmrahmens (49) am Boden (50) oder auf bzw. an den Anbauwänden befestigt werden.

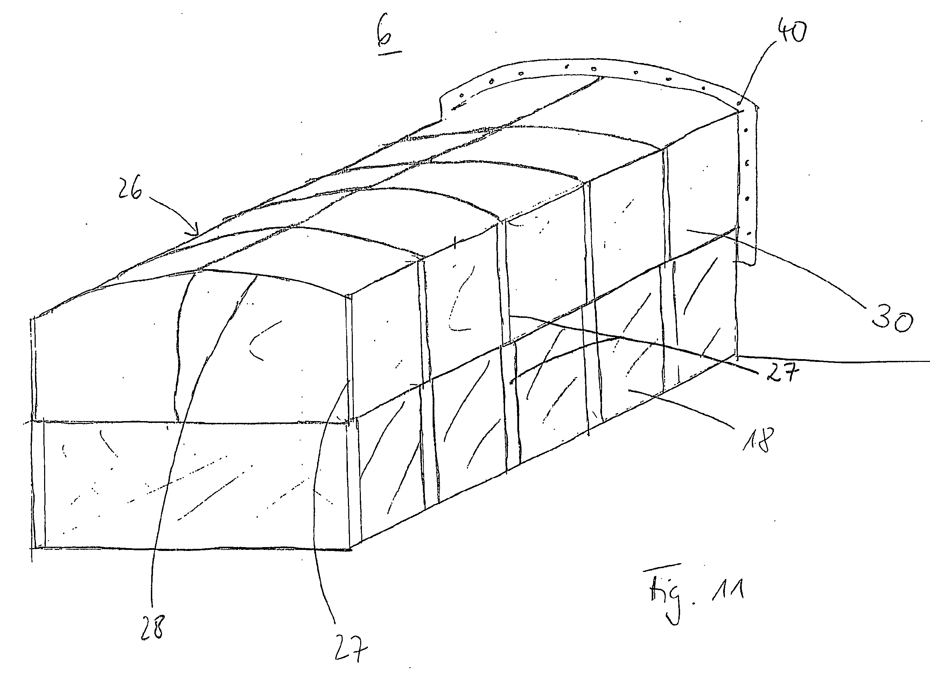

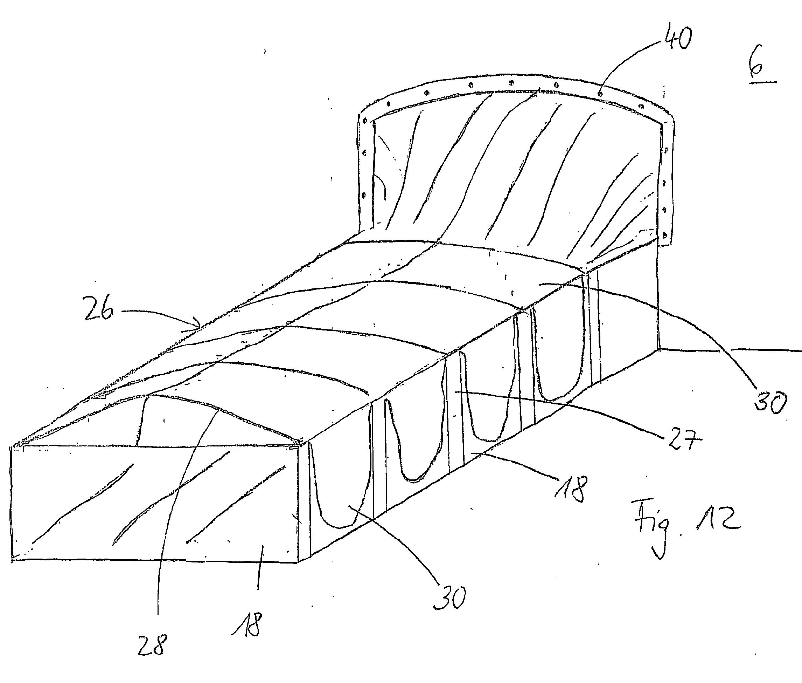

Die Figuren 11 und 12 zeigen eine Abfallbehandlungsanlage (1) mit einem Behandlungsraum (16), der eine Hebe- und Senkvorrichtung (20) in Form einer mechanischen Höhenverstellkonstruktion (26) aufweist. Vorzugsweise besteht diese konstruktion (26) aus Metallen, insbesondere Edelstahl. Dazu sind an den Anfahrwänden (18) teleskopartig auseinanderziehbare Träger (27) befestigt. Die Träger (27) sind mit Stützstäben (28) zu einem in der Höhe verstellbaren Gestell verbunden. An der Innenseite oder Außenseite des Gestells ist die Abdeckplane (30) befestigt, welche den Abfallbehandlungraum (16) bildet. In Figur 11 sind die Träger (27) auseinandergezogen, die Abdeckplane (30) ist angehoben und befindet sich mit Abstand über der Abfallmiete. Der Abdeckplanenrand (32) ist mit der oben beschriebenen Befestigungsvorrichtung (40) an der Hallenwand (6) und den Anbauwänden (18) starr und luftdicht befestigt. In Figur 12 sind die Träger (27) ineinandergeschoben, der Abfallbehandlungsraum (16) ist in einem gesenkten Zustand so dass die Abdeckplane (30) auf der Abfallmiete liegt und den aeroben Behandlungsprozess beschleunigt.

## Patentansprüche

1. Abfallbehandlungsanlage (1), mit
a) einer Halle (5) mit zumindest einer Hallenwand (6), einem Hallendach (9) und mit einem Zugang (12) zum Einführen und Entfernen von Abfall (2),
b) mit einer Be- und Entlüftungsanlage (14) zur Zu- und Abfuhr von Luft in und aus der Halle (5),
c) mit mindestens einem an einer Außenseite der Hallenwand (6) angeordneten Abfallbehandlungsraum (16) und
d) mit einer die Halle (5) mit dem Abfallbehandlungsraum (16) verbindenden Hallenwandöffnung (7) zum Einführen des Abfalls (2) aus der Halle (5) in den Abfallbehandlungsraum (16) und zum Entfernen des behandelten Abfalls aus dem Abfallbehandlungsraum (16) in die Halle (5),
e) der Abfallbehandlungsraum (16) wird durch eine mit einer Hebe- und Senkvorrichtung (20) versehenen Abdeckplane (30) mit einem Abdeckplanenrand (32) gebildet, wobei der Abdeckplanenrand (32) zur Hallenwand (6) und zum Boden mittels einer Befestigungsvorrichtung (40) starr und luftdicht angeordnet ist.

2. Anlage (1) nach Anspruch 1, wobei der Abfallbehandlungsraum (16) Anbauwände (18) aufweist, die bodenseitig den Abfallbehandlungsraum (16) begrenzen und der Abdeckplanenrand (32) zur Hallenwand (6) und zu den Anbauwänden (18) starr und luftdicht angeordnet ist.

3. Anlage (1) nach Anspruch 1, wobei die Befestigungsvorrichtung (40) mindestens ein anpassungsfähiges Material (42) aufweist.

4. Anlage (1) nach Anspruch 3, wobei das Material (42) aus der Materialgruppe enthaltend flexible Materialien wie Schaumstoff, Gummi, beschichtete Trägerstoffe und Polymerstreifen ausgewählt ist.

5. Anlage (1) nach Anspruch 3, wobei das Material (42) ein beschichtetes Textil aufweist.

6. Anlage (1) nach Anspruch 3, wobei das Material (42) jeweils zwischen dem Abdeckplanenrand (32) und der Hallenwand (6) sowie zwischen dem Abdeckplanenrand (32) und dem Boden (50) angeordnet ist.

7. Anlage (1) nach Anspruch 2 und 3, wobei das Material (42) jeweils zwischen dem Abdeckplanenrand (32) und der Hallenwand (6) sowie zwischen dem Abdeckplanenrand (32) und den Anbauwänden (18) angeordnet ist.

8. Anlage (1) nach Anspruch 1, wobei die Befestigungsvorrichtung (40) mindestens einen Klemmrahmen (48) aufweist, in welchen der Abdeckplanenrand (32) geklemmt ist.

9. Anlage (1) nach Anspruch 1, wobei die Befestigungsvorrichtung (40) Fixierelemente wie Schrauben, Nägel, Klemmen, Nieten umfaßt.

10. Anlage (1) nach Anspruch 1, wobei der Abfallbehandlungsraum (16) zur Umgebung partikeldicht ist.

11. Anlage (1) nach Anspruch 1, wobei die Abdeckplane (30) eine wasserdichte und gasdurchlässige Membrane (34) aufweist.

12. Anlage (1) nach Anspruch 11, wobei die Membrane (34) porös ist.

13. Anlage (1) nach Anspruch 11, wobei die Membrane (34) aus der Gruppe der Polyolefine, Polyester, Polyvinylchloride, Polyurethane oder Fluorpolymere ausgewählt ist.

14. Anlage (1) nach Anspruch 11, wobei die Membrane (34) gerecktes Polytetrafluorethylen (ePTFE) aufweist.

15. Anlage (1) nach Anspruch 11, wobei die Membrane (34) mit mindestens einer textilen Schicht (36) verbunden ist.

16. Anlage (1) nach Anspruch 15, wobei die textile Schicht (36) Polyester, Polyamid, Polyethylen, Polyacrylat, Polypropylen, Glasfaser oder Fluorpolymer aufweist.

17. Anlage (1) nach Anspruch 11, wobei die Abdeckplane (30) eine Luftdurchlässigkeit zwischen 3 und 100m³/m²/h bei 200 Pa Druckdifferenz aufweist.

18. Anlage (1) nach Anspruch 1, wobei die Hebe- und Senkvorrichtung (20) eine pneumatische Stützkonstruktion (22) oder eine mechanische Höhenverstellkonstruktion (26) umfaßt.

19. Anlage (1) nach Anspruch 18, wobei die pneumatische Stützkonstruktion (22) eine Anzahl aufblasbarer Schläuche (23) vorsieht.

20. Anlage (1) nach Anspruch 19, wobei die Schläuche (23) einem Druck von mindestens 10kPa standhalten.

21. Anlage (1) nach Anspruch 18, wobei die pneumatische Stützkonstruktion (22) eine Anzahl von mit Flüssigkeit füllbarer Schläuche vorsieht.

22. Anlage (1) nach Anspruch 18, wobei die mechanische Höhenverstellkonstruktion (26) in der Höhe verstellbare Träger (27) aufweist.

23. Anlage (1) nach Anspruch 22, wobei die Träger (27) teleskopartig auseinanderziehbar sind.

24. Anlage (1) nach Anspruch 1, wobei im Boden der Halle (5) eine Anzahl von ersten Bodenöffnungen (52) vorgesehen sind und die Be- und Entlüftungsanlage (14) über die Bodenöffnungen (52) Luft aus dem Inneren der Halle (5) abführt.

25. Anlage (1) nach Anspruch 1 und 24, wobei im Boden des Inneren des Abfallbehandlungsraumes (16) eine Anzahl von zweiten Bodenöffnungen (54) vorgesehen sind und die Be- und Entlüftungsanlage (14) die abgeführte Luft aus der Halle (5) über die zweiten Bodenöffnungen (54) dem Inneren des Abfallbehandlungsraumes (16) zuführt.

26. Anlage (1) nach Anspruch 1, wobei die Hallenwandöffnung (7) verschließbar ist.

27. Anlage (1) nach Anspruch 1, wobei die Be- und Entlüftungsanlage (14) mindestens einen Abluftfilter (3) aufweist.

## Claims

1. Waste treatment installation (1), with
a) a hall (5) with at least one hall wall (6), a hall roof (9) and with an access (12) for introducing and removing waste (2),
b) with a ventilating and venting installation (14) for supplying and removing air into and from the hall (5),
c) with at least one waste treatment space (16), arranged on an outer side of the hall wall (6), and
d) with a hall wall opening (7), which connects the hall (5) to the waste treatment space (16) and serves for introducing the waste (2) from the hall (5) into the waste treatment space (16) and for removing the treated waste from the waste treatment space (16) into the hall (5),
e) the waste treatment space (16) is formed by a cover tarpaulin (30) provided with a raising and lowering device (20) and having a cover tarpaulin edge (32), the cover tarpaulin edge (32) being arranged in a rigid and airtight manner with respect to the hall wall (6) and with respect to the ground by means of a fastening device (40).

2. Installation (1) according to Claim 1, the waste treatment space (16) having add-on walls (18) which delimit the waste treatment space (16) at the bottom, and the cover tarpaulin edge (32) being arranged in a rigid and airtight manner with respect to the hall wall (6) and with respect to the add-on walls (18).

3. Installation (1) according to Claim 1, the fastening device (40) comprising at least one adaptable material (42).

4. Installation (1) according to Claim 3, the material (42) being selected from the material group including flexible materials such as foam, rubber, coated backing materials and polymer strips.

5. Installation (1) according to Claim 3, the material (42) comprising a coated textile.

6. Installation (1) according to Claim 3, the material (42) respectively being arranged between the cover tarpaulin edge (32) and the hall wall (6) and between the cover tarpaulin edge (32) and the ground (50).

7. Installation (1) according to Claims 2 and 3, the material (42) respectively being arranged between the cover tarpaulin edge (32) and the hall wall (6) and between the cover tarpaulin edge (32) and the add-on walls (18).

8. Installation (1) according to Claim 1, the fastening device (40) having at least one clamping frame (48), into which the cover tarpaulin edge (32) is clamped.

9. Installation (1) according to Claim 1, the fastening device (40) comprising fixing elements such as screws, nails, clips or rivets.

10. Installation (1) according to Claim 1, the waste treatment space (16) being impermeable to particles with respect to the surroundings.

11. Installation (1) according to Claim 1, the cover tarpaulin (30) having a waterproof and gas-permeable membrane (34).

12. Installation (1) according to Claim 11, the membrane (34) being porous.

13. Installation (1) according to Claim 11, the membrane (34) being selected from the group comprising polyolefins, polyesters, polyvinyl chloride, polyurethanes or fluoropolymers.

14. Installation (1) according to Claim 11, the membrane (34) comprising expanded polytetrafluoroethylene (ePTFE).

15. Installation (1) according to Claim 11, the membrane (34) being joined to at least one textile layer (36).

16. Installation (1) according to Claim 15, the textile layer (36) comprising polyester, polyamide, polyethylene, polyacrylate, polypropylene, glass fiber or fluoropolymer.

17. Installation (1) according to Claim 11, the cover tarpaulin (30) having an air permeability of between 3 and 100 m3/m²/h under a pressure difference of 200 Pa.

18. Installation (1) according to Claim 1, the raising and lowering device (20) comprising a pneumatic supporting construction (22) or a mechanical height-adjusting construction (26).

19. Installation (1) according to Claim 18, the pneumatic supporting construction (22) providing a number of inflatable tubes (23).

20. Installation (1) according to Claim 19, the tubes (23) withstanding a pressure of at least 10 kPa.

21. Installation (1) according to Claim 18, the pneumatic supporting construction (22) providing a number of tubes which can be filled with liquid.

22. Installation (1) according to Claim 18, the mechanical height-adjusting construction (26) having carriers (27) which are adjustable in height.

23. Installation (1), according to Claim 22, the carriers (27) being telescopically extendable.

24. Installation (1) according to Claim 1, a number of first floor openings (52) being provided in the floor of the hall (5) and the ventilating and venting installation (14) removing air from the interior of the hall (5) via the floor openings (52).

25. Installation (1) according to Claims 1 and 24, a number of second floor openings (54) being provided in the floor of the interior of the waste treatment space (16), and the ventilating and venting installation (14) passing the air removed from the hall (5) to the interior of the waste treatment space (16) via the second floor openings (54).

26. Installation (1) according to Claim 1, the hall wall opening (7) being closable.

27. Installation (1) according to Claim 1, the ventilating and venting installation (14) having at least one foul-air filter (3).

## Revendications

1. Unité de traitement des déchets (1), comprenant
a) un hall (5), avec au moins une paroi de hall (6), un toit de hall (9) et avec un accès (12) pour introduire et pour évacuer des déchets (2),
b) une unité d'aération et de ventilation (14) pour alimenter le hall en air et pour évacuer l'air hors du hall (5),
c) au moins un espace de traitement des déchets (16) disposé sur la face extérieure de la paroi de hall (6) et
d) une ouverture dans la paroi du hall (7) reliant le hall (5) et l'espace de traitement des déchets (16) pour introduire les déchets (2) à partir du hall (5) dans l'espace de traitement des déchets (16) et pour évacuer les déchets traités hors de l'espace de traitement des déchets (16) vers le hall (5),
e) l'espace de traitement des déchets (16) étant formé par une bâche de recouvrement (30) munie d'un dispositif de relèvement et d'abaissement (20), avec un bord de bâche de recouvrement (32), le bord de la bâche de recouvrement (32) étant disposé de façon rigide et étanche à l'air par rapport à la paroi de hall (6) et au fond, au moyen d'un dispositif de fixation (40).

2. Unité (1) selon la revendication 1, l'espace de traitement des déchets (16) comportant des parois rajoutées (18), qui délimitent l'espace de traitement des déchets (16) côté fond et le bord de la bâche de recouvrement (32). étant disposé de façon rigide et étanche à l'air par rapport à la paroi de hall (6) et aux parois rajoutées (18).

3. Unité (1) selon la revendication 1, le dispositif de fixation (40) comportant au moins un matériau adaptable (42).

4. Unité (1) selon la revendication 3, le matériau (42) étant choisi dans le groupe de matériaux comprenant des matériaux flexibles, comme la mousse synthétique, le caoutchouc, des substances porteuses enduites et des bandes de polymère.

5. Unité (1) selon la revendication 3, le matériau (42) comportant un textile enduit.

6. Unité (1) selon la revendication 3, le matériau (42) étant respectivement disposé entre le bord de la bâche de recouvrement (32) et la paroi de hall (6), et entre le bord de la bâche de recouvrement (32) et le fond (50).

7. Unité (1) selon les revendications 2 et 3, le matériau (42) étant disposé respectivement entre le bord de la bâche de recouvrement (32) et la paroi de hall (6) et entre le bord de la bâche de recouvrement (32) et les parois rajoutées (18).

8. Unité (1) selon la revendication 1, le dispositif de fixation (40) comportant au moins un cadre de serrage (48), dans lequel est serré le bord de la bâche de recouvrement (32).

9. Unité (1) selon la revendication 1, le dispositif de fixation (40) comprenant des éléments de fixation, tels que des vis, des clous, des agrafes, des rivets.

10. Unité (1) selon la revendication 1, l'espace de traitement des déchets (16) étant étanche aux particules, par rapport à l'environnement.

11. Unité (1) selon la revendication 1, la bâche de recouvrement (30) comportant une membrane (34) étanche à l'eau et perméable au gaz.

12. Unité (1) selon la revendication 11, la membrane (34) étant poreuse.

13. Unité (1) selon la revendication 11, la membrane (34) étant choisie dans le groupe des polyoléfines, des polyesters, des chlorures de polyvinyle, des polyuréthanes ou des polymères fluorés.

14. Unité (1) selon la revendication 11, la membrane (34) comportant du polytétrafluoroéthylène étiré (ePTFE).

15. Unité (1) selon la revendication 11, la membrane (34) étant reliée avec au moins une couche textile (36).

16. Unité (1) selon la revendication 15, la couche textile (36) comportant du polyester, du polyamide, du polyéthylène, du polyacrylate, du polypropylène, des fibres de verre ou un polymère fluoré.

17. Unité (1) selon la revendication 11, la bâche de recouvrement (30) présentant une perméabilité à l'air comprise entre 3 et 100 m³/m²/h, pour une pression différentielle de 2.00 Pa.

18. Unité (1) selon la revendication 1, le dispositif de relèvement et d'abaissement (20) comprenant une structure d'appui pneumatique (22) ou une structure mécanique de réglage en hauteur (26).

19. Unité (1) selon la revendication 18, la structure d'appui pneumatique (22) prévoyant un certain nombre de flexibles (23) gonflables.

20. Unité (1) selon la revendication 19, les flexibles (23) résistant à une pression d'au moins 10 kPa.

21. Unité (1) selon la revendication 18, la structure d'appui pneumatique (22) prévoyant un certain nombre de flexibles, susceptibles d'être remplis de liquide.

22. Unité (1) selon la revendication 18, la structure mécanique de réglage en hauteur (26) comportant des supports (27) réglables en hauteur.

23. Unité (1) selon la revendication 22, les supports (27) étant télescopiquement extensibles.

24. Unité (1) selon la revendication 1, un certain nombre de premières ouvertures dans le fond (52) étant prévu dans le fond du hall (5) et l'unité d'aération et de ventilation (14) évacuant de l'air à partir de l'intérieur du hall (5) par l'intermédiaire des ouvertures dans le fond (52).

25. Unité (1) selon les revendications 1 et 24, un certain nombre de deuxièmes ouvertures dans le fond (54) étant prévu dans le fond de l'intérieur de l'espace de traitement des déchets (16) et l'unité d'aération et de ventilation (14) alimentant l'air évacué à partir du hall (5) vers l'intérieur de l'espace de traitement des déchets (16) par l'intermédiaire des deuxièmes ouvertures dans le fond (54).

26. Unité (1) selon la revendication 1, l'ouverture dans la paroi du hall (7) pouvant se fermer.

27. Unité (1) selon la revendication 1, l'unité d'aération et de ventilation (14) comportant au moins un filtre d'évacuation d'air (3).
